# EUROPEAN PATENT SPECIFICATION

(11) **EP 2 220 085 B1**
(45) Date of publication and mention of the grant of the patent: **12.02.2014**
(21) Application number: 08856745.8
(22) Date of filing: 03.12.2008
(51) Int. Cl.: C07D 471/04, C07D 455/06, A61K 31/4353

(54) **FLUOROALKYL TETRABENAZINE CARBINOL COMPOUNDS AS IMAGING AGENTS AND PROBES**
FLUORALKYLTETRABENAZINCARBINOLVERBINDUNGEN ALS BILDDARSTELLENDE MITTEL UND SONDEN
COMPOSES DE METHANOL DE FLUOROALKYLTETRABENAZINE EN TANT QU'AGENTS D'IMAGERIE ET SONDES

(30) Priority: 07.12.2007 US 952340; 07.12.2007 US 952325
(43) Date of publication of application: 25.08.2010
(73) Proprietor: General Electric Company, Schenectady, NY 12345 (US)
(72) Inventor: AMARASINGHE, Kande Kankananamalage Dayarathna, Latham, New York 12110 (US); RISHEL, Michael James, Saratoga Springs, NY12866 (US); DINN, Sean Richard, Delmar, New York 12054 (US); JOHNSON, Bruce Fletcher, Scotia, New York 12302 (US)
(74) Representative: Canning, Lewis R.
(86) International application number: PCT/US2008/085304
(87) International publication number: WO 2009/073677

(56) References cited:
- WO-A-2005/077946
- ZHENG, FANG ET AL: "Computational neural network analysis of the affinity of lobeline and tetrabenazine analogs for the vesicular monoamine transporter-2" BIOORGANIC & MEDICINAL CHEMISTRY , 15(8), 2975-2992 CODEN: BMECEP; ISSN: 0968-0896, 11 February 2007 (2007-02-11), XP002515369
- GOSWAMI R ET AL: "Fluoroalkyl derivatives of dihydrotetrabenazine as positron emission tomography imaging agents targeting vesicular monoamine transporters" NUCLEAR MEDICINE AND BIOLOGY, ELSEVIER, NY, US, vol. 33, no. 6, 1 August 2006 (2006-08-01), pages 685-694, XP025103516 ISSN: 0969-8051 [retrieved on 2006-08-01] cited in the application
- KILBOURN ET AL: "Pharmacokinetics of [<18>F]fluoroalkyl derivatives of dihydrotetrabenazine in rat and monkey brain" NUCLEAR MEDICINE AND BIOLOGY, ELSEVIER, NY, US, vol. 34, no. 3, 23 March 2007 (2007-03-23), pages 233-237, XP005935419 ISSN: 0969-8051

## Description

### BACKGROUND

This invention relates to carbinol compounds related to tetrabenazine and intermediates useful in the preparation of such fluoroalkyl tetrabenazine carbinol compounds

Since first reported on in 1957 (Pletscher, A. (1957) Release of 5-hydioxytryptamine by benzoquinolizine derivatives with sedative action, Science 126, 507), tetrabenazine and structurally related compounds have been widely investigated, and a number of tetrabenazine (TBZ) compounds and derivatives of tetrabenazine have shown promise in the treatment of a variety of conditions affecting human health. For example, dihydrotetrabenazine has been identified as an agent for the treatment of schizophrenia and other psychoses (See for example WO 2007017654 A1), and tetrabenazine has shown promise as an agent in the treatment of Huntington's disease (Neurology (2006), 66(3), 366-372). Although most preparations used in biological studies of tetrabenazine and its derivatives have been carried out on racemates, in at least one instance the biological activity exhibited by enantiomers tested separately was highly differentiated (See Koeppe, R. A. et al. (1999) Assessment of extrastriatal vesicular monoamine transporter binding site density using stereoisomers of [11C] dihydi-otetrabenazine, J Cereb Blood Flow Metab 19, 1376-1384).

More recently, derivatives of 9-desmethyl (±)-dihydrotetrabenazine incorporating a fluorine-18 atom have been shown to be useful as PET imaging agents, Nuclear Medicine and Biology 33 (2006) 685-694. See also Nuclear Medicine and Biology 34 (2007) 239-246; and Nuclear Medicine and Biology 34 (2007) 233-237.

The present invention provides both a new class of fluorinated tetrabenazine derivatives and fluorinated tetrabenazine analogs, and discloses efficient synthetic methodology, which may be used to prepare such compounds in enantiomerically enriched or racemic forms. The fluoroalkyl tetrabenazine carbinol compounds provided by the present invention are useful as PET imaging agents, probes for the development of PET imaging agents, and therapeutic agents. In addition, the present invention provides novel synthetic intermediate compositions, which may be used to prepare either or both enantiomers of the subject tetrabenazine derivatives.

### BRIEF DESCRIPTION

In one embodiment, the present invention provides a fluoroalkyl tetrabenazine carbinol compound having structure I 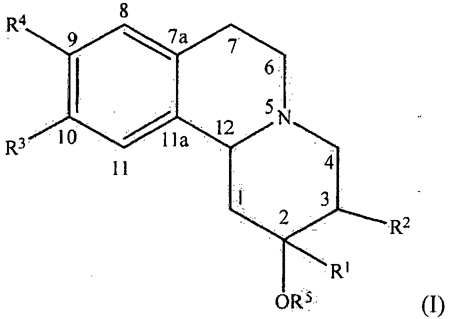 wherein
R¹ is selected from C₁₋₆fluoroalkyl, C₂₋₆fluoroalkenyl, C₂₋₆fluoroalkynyl C₁₋₆fluoroalkoxy(C₁₋₆alkyl), C₁₋₆fluorohaloalkyl, C₁₋₆fluorohydroxyalkyl, and C₁₋₆fluoroalkylcarbonyl(C₁₋₆alkyl), and is suitably C₂₋₆fluoroalkynyl;
R² is selected from C₁₋₆alkyl and C₃₋₈cycloalkyl, and is suitably C₁₋₆alkyl;
R³ and R⁴ are each independently selected from C₁₋₆alkyl and C₁₋₆alkoxy;
R⁵ is selected from hydrogen, (C₁₋₆alkyl)carbonyl, and phenyl.

In another embodiment, the present invention provides a PET imaging agent comprising a fluoroalkyl tetrabenazine carbinol compound having structure I 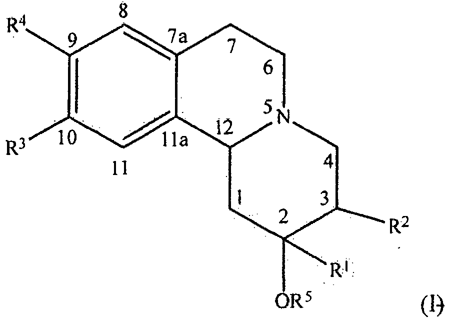 further comprising a fluorine-18 atom.

In yet another embodiment, the present invention provides fluoroalkyl tetrabenazine carbinol compounds having structure IV 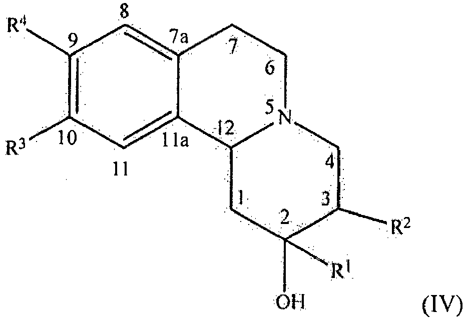 wherein R¹; R²; R³ and R⁴ are as defined for structure I.

In yet another embodiment, the present invention provides a PET imaging agent further comprising a fluorine-18 atom.

### DETAILED DESCRIPTION

In the following specification and the claims, which follow, reference will be made to a number of terms, which shall be defined to have the following meanings.

"Optional" or "optionally" means that the subsequently described event or circumstance may or may not occur, and that the description includes instances where the event occurs and instances where it does not.

As used herein, the term "solvent" can refer to a single solvent or a mixture of solvents.

Approximating language, as used herein throughout the specification and claims, may be applied to modify any quantitative representation that could permissibly vary without resulting in a change in the basic function to which it is related. Accordingly, a value modified by a term or terms, such as "about", is not to be limited to the precise value specified. In some instances, the approximating language may correspond to the precision of an instrument for measuring the value.

As used herein, the term "aromatic radical" refers to an array of atoms having a valence of at least one comprising at least one aromatic group. The array of atoms having a valence of at least one comprising at least one aromatic group may include heteroatoms such as nitrogen, sulfur, selenium, silicon and oxygen, or may be composed exclusively of carbon and hydrogen. As used herein, the term "aromatic radical" includes but is not limited to phenyl, pyridyl, furanyl, thienyl, naphthyl, phenylene, and biphenyl radicals. As noted, the aromatic radical contains at least one aromatic group. The aromatic group is invariably a cyclic structure having 4n+2 "delocalized" electrons where "n" is an integer equal to 1 or greater, as illustrated by phenyl groups (n = 1), thienyl groups (n = 1), furanyl groups (n = 1), naphthyl groups (n = 2), azulenyl groups (n = 2), anthraceneyl groups (n = 3) and the like. The aromatic radical may also include nonaromatic components. For example, a benzyl group is an aromatic radical, which comprises a phenyl ring (the aromatic group) and a methylene group (the nonaromatic component). Similarly a tetrahydronaphthyl radical is an aromatic radical comprising an aromatic group (C₆H₃) fused to a nonaromatic component -(CH₂)₄-. For convenience, the term "aromatic radical" is defined herein to encompass a wide range of functional groups such as alkyl groups, alkenyl groups, alkynyl groups, haloalkyl groups, haloaromatic groups, conjugated dienyl groups, alcohol groups, ether groups, aldehyde groups, ketone groups, carboxylic acid groups, acyl groups (for example carboxylic acid derivatives such as esters and amides), amine groups, nitro groups, and the like. For example, the 4-methylphenyl radical is a C₇ aromatic radical comprising a methyl group, the methyl group being a functional group which is an alkyl group. Similarly, the 2-nitrophenyl group is a C₆ aromatic radical comprising a nitro group, the nitro group being a functional group. Aromatic radicals include halogenated aromatic radicals such as 4-trifluoromethylphenyl, hexafluoroisopropylidenebis(4-phen-1-yloxy) (i.e., -OPhC(CF₃)₂PhO-), 4-chloromethylphen-1-yl, 3-trifluorovinyl-2-thienyl, 3-trichloromethylphen-1-yl (i.e., 3-CCl₃Ph-), 4-(3-bromoprop-1-yl)phen-1-yl (i.e., 4-BrCH₂CH₂CH₂Ph-), and the like. Further examples of aromatic radicals include 4-allyloxyphen-1-oxy, 4-aminophen-1-yl (i.e., 4-H₂NPh-), 3-aminocarbonylphen-1-yl (i.e., NH₂COPh-), 4-benzoylphen-1-yl, dicyanomethylidenebis(4-phen-1-yloxy) (i.e., -OPhC(CN)₂PhO-), 3-methylphen-1-yl, methylenebis(4-phen-1-yloxy) (i.e., -OPhCH₂PhO-), 2-ethylphen-1-yl, phenylethenyl, 3-formyl-2-thienyl, 2-hexyl-5-furanyl, hexamethylene-1,6-bis(4-phen-1-yloxy) (i.e., -OPh(CH₂)₆PhO-), 4-hydroxymethylphen-1-yl (i.e., 4-HOCH₂Ph-), 4-mercaptomethylphen-1-yl (i.e., 4-HSCH₂Ph-), 4-methylthiophen-1-yl (i.e., 4-CH₃SPh-), 3-methoxyphen-1-yl, 2-methoxycarbonylphen-1-yloxy (e.g., methyl salicyl), 2-nitromethylphen-1-yl (i.e., 2-NO₂CH₂Ph), 3-trimethylsilylphen-1-yl, 4-t-butyldimethylsilylphenl-1-yl, 4-vinylphen-1-yl, vinylidenebis(phenyl), and the like. The term "a C₃-C₁₀ aromatic radical" includes aromatic radicals containing at least three but no more than 10 carbon atoms. The aromatic radical 1-imidazolyl (C₃H₂N₂-) represents a C₃ aromatic radical. The benzyl radical (C₇H₇-) represents a C₇ aromatic radical.

As used herein the term "cycloaliphatic radical" refers to a radical having a valence of at least one, and comprising an array of atoms which is cyclic but which is not aromatic. As defined herein a "cycloaliphatic radical" does not contain an aromatic group. A "cycloaliphatic radical" may comprise one or more noncyclic components. For example, a cyclohexylmethyl group (C₆H₁₁CH₂-) is a cycloaliphatic radical, which comprises a cyclohexyl ring (the array of atoms which is cyclic but which is not aromatic) and a methylene group (the noncyclic component). The cycloaliphatic radical may include heteroatoms such as nitrogen, sulfur, selenium, silicon and oxygen, or may be composed exclusively of carbon and hydrogen. For convenience, the term "cycloaliphatic radical" is defined herein to encompass a wide range of functional groups such as alkyl groups, alkenyl groups, alkynyl groups, haloalkyl groups, conjugated dienyl groups, alcohol groups, ether groups, aldehyde groups, ketone groups, carboxylic acid groups, acyl groups (for example carboxylic acid derivatives such as esters and amides), amine groups, nitro groups, and the like. For example, the 4-methylcyclopent-1-yl radical is a C₆ cycloaliphatic radical comprising a methyl group, the methyl group being a functional group which is an alkyl group. Similarly, the 2-nitrocyclobut-1-yl radical is a C₄ cycloaliphatic radical comprising a nitro group, the nitro group being a functional group. A cycloaliphatic radical may comprise one or more halogen atoms which may be the same or different. Halogen atoms include, for example; fluorine, chlorine, bromine, and iodine. Cycloaliphatic radicals comprising one or more halogen atoms include 2-trifluoromethylcyclohex-1-yl, 4-bromodifluoromethylcyclooct-1-yl, 2-chlorodifluoromethylcyclohex-1-yl, hexafluoroisopropylidene-2,2-bis (cyclohex-4-yl) (i.e., -C₆H₁₀C(CF₃)₂ C₆H₁₀-), 2-chloromethylcyclohex-1-yl, 3- difluoromethylenecyclohex-1-yl, 4-trichloromethylcyclohex-1-yloxy, 4-bromodichloromethylcyclohex-1-ylthio, 2-bromoethylcyclopent-1-yl, 2-bromopropylcyclohex-1-yloxy (e.g., CH₃CHBrCH₂C₆H₁₀O-), and the like. Further examples of cycloaliphatic radicals include 4-allyloxycyclohex-1-yl, 4-aminocyclohex-1-yl (i.e., H₂NC₆H₁₀-), 4-aminocarbonylcyclopent-1-yl (i.e., NH₂COC₅H₈-), 4-acetyloxycyclohex-1-yl, 2,2-dicyanoisopropylidenebis(cyclohex-4-yloxy) (i.e., -OC₆H₁₀C(CN)₂C₆H₁₀O-), 3-methylcyclohex-1-yl, methylenebis(cyclohex-4-yloxy) (i.e., -OC₆H₁₀CH₂C₆H₁₀O-), 1-ethylcyclobut-1-yl, cyclopropylethenyl, 3-formyl-2-terahydrofuranyl, 2-hexyl-5-tetrahydrofuranyl, hexamethylene-1,6-bis(cyclohex-4-yloxy) (i.e., -O C₆H₁₀(CH₂)₆C₆H₁₀O-), 4-hydroxymethylcyclohex-1-yl (i.e., 4-HOCH₂C₆H₁₀-), 4-mercaptomethylcyclohex-1-yl (i.e., 4-HSCH₂C₆H₁₀-), 4-methylthiocyclohex-1-yl (i.e., 4-CH₃SC₆H₁₀-), 4-methoxycyclohex-1-yl, 2-methoxycarbonylcyclohex-1-yloxy (2-CH₃OCOC₆H₁₀O-), 4-nitromethylcyclohex-1-yl (i.e., NO₂CH₂C₆H₁₀-), 3-trimethylsilylcyclohex-1-yl, 2-t-butyldimethylsilylcyclopent-1-yl, 4-trimethoxysilylethylcyclohex-1-yl (e.g., (CH₃O)₃SiCH₂CH₂C₆H₁₀-), 4-vinylcyclohexen-1-yl, vinylidenebis(cyclohexyl), and the like. The term "a C₃-C₁₀ cycloaliphatic radical" includes cycloaliphatic radicals containing at least three but no more than 10 carbon atoms. The cycloaliphatic radical 2-tetrahydrofuranyl (C₄H₇O-) represents a C₄ cycloaliphatic radical. The cyclohexylmethyl radical (C₆H₁₁CH₂-) represents a C₇ cycloaliphatic radical.

As used herein the term "aliphatic radical" refers to an organic radical having a valence of at least one consisting of a linear or branched array of atoms, which is not cyclic. Aliphatic radicals are defined to comprise at least one carbon atom. The array of atoms comprising the aliphatic radical may include heteroatoms such as nitrogen, sulfur, silicon, selenium and oxygen or may be composed exclusively of carbon and hydrogen. For convenience, the term "aliphatic radical" is defined herein to encompass, as part of the "linear or branched array of atoms which is not cyclic" a wide range of functional groups such as alkyl groups, alkenyl groups, alkynyl groups, haloalkyl groups, conjugated dienyl groups, alcohol groups, ether groups, aldehyde groups, ketone groups, carboxylic acid groups, acyl groups (for example carboxylic acid derivatives such as esters and amides), amine groups, nitro groups, and the like. For example, the 4-methylpent-1-yl radical is a C₆ aliphatic radical comprising a methyl group, the methyl group being a functional group which is an alkyl group. Similarly, the 4-nitrobut-1-yl group is a C₄ aliphatic radical comprising a nitro group, the nitro group being a functional group. An aliphatic radical may be a haloalkyl group which comprises one or more halogen atoms which may be the same or different. Halogen atoms include, for example; fluorine, chlorine, bromine, and iodine. Aliphatic radicals comprising one or more halogen atoms include the alkyl halides trifluoromethyl, bromodifluoromethyl, chlorodifluoromethyl, hexafluoroisopropylidene, chloromethyl, difluorovinylidene, trichloromethyl, bromodichloromethyl, bromoethyl, 2-bromotrimethylene (e.g., -CH₂CHBrCH₂-), and the like. Further examples of aliphatic radicals include allyl, aminocarbonyl (i.e.,-CONH₂), carbonyl, 2,2-dicyanoisopropylidene (i.e., -CH₂C(CN)₂CH₂-), methyl (i.e.,-CH₃), methylene (i.e., -CH₂-), ethyl, ethylene, formyl (i.e.,-CHO), hexyl, hexamethylene, hydroxymethyl (i.e., -CH₂OH), mercaptomethyl (i.e., -CH₂SH), methylthio (i.e., -SCH₃), methylthiomethyl (i.e., -CH₂SCH₃), methoxy, methoxycarbonyl (i.e., CH₃OCO-), nitromethyl (i.e., -CH₂NO₂), thiocarbonyl, trimethylsilyl (i.e., (CH₃)₃Si-), t-butyldimethylsilyl, 3-trimethyoxysilylpropyl (i.e., (CH₃O)₃SiCH₂CH₂CH₂-), vinyl, vinylidene, and the like. By way of further example, a C₁-C₁₀ aliphatic radical contains at least one but no more than 10 carbon atoms. A methyl group (i.e., CH₃-) is an example of a C₁ aliphatic radical. A decyl group (i.e., CH₃(CH₂)₉-) is an example of a C₁₀ aliphatic radical.

As noted, in one embodiment the present invention provides a fluoroalkyl tetrabenazine carbinol compound having structure I

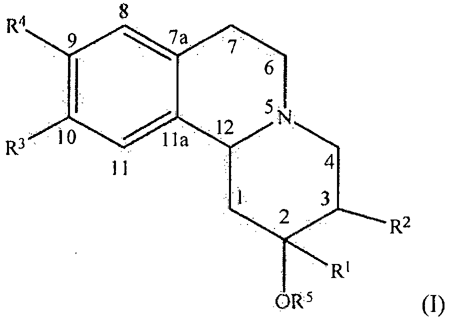

As noted, in another embodiment the present invention provides a fluoroalkyl tetrabenazine carbinol compound having structure IV

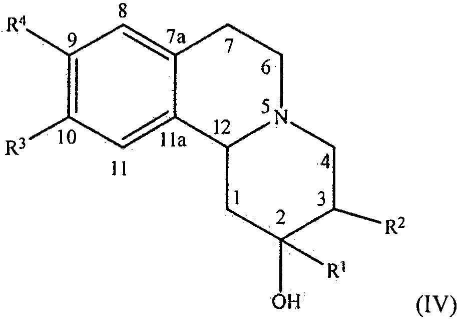

Those skilled in the art will appreciate that the term "fluoroalkyl tetrabenazine carbinol compound" refers to compounds falling within the scope of generic structure 1. For convenience, compounds defined by generic structure I are referred to at times herein as "tetrabenazine carbinol compounds".

In the compounds of formulae (I) and (IV) and in other aspects of the invention below:
R¹ is suitably selected from C₁₋₆fluoroalkyl, C₂₋₆fluoroalkenyl, C₂₋₆fluoroalkynyl C₁₋₆fluoroalkoxy(C₁₋₆alkyl), C₁₋₆fluorohaloalkyl, C₁₋₆fluorohydroxyalkyl, and C₁₋₆fluoroalkylcarbonyl(C₁₋₆alkyl). In one aspect of the invention R¹ is C₂₆fluoroalkynyl.
R² is suitably selected from C₁₋₆alkyl and C₃₋₈cycloalkyl and is more suitably C₁₆alkyl;
R³ and R⁴ are each suitably independently selected from C₁₋₆alkyl and C₁₋₆alkoxy.

In the compounds of formula (I) and in related aspects of the invention below, R⁵ is suitably selected from hydrogen, (C₁₋₆alkyl)carbonyl, and phenyl. In one aspect R⁵ is hydrogen. In a separate aspect R⁵ is (C₁₋₆alkyl)carbonyl, more suitably CH₃C(O)-.

The fluoroalkyl tetrabenazine carbinol compounds provided by the present invention are shown herein to possess a high affinity for Type 2 Vesicular Monoamine Transporters (VMAT-2), a group of biomarkers, which correlate *inter alia* with diabetic activity in human patients. The discovery that substitution at ring position-2 of the tetrabenazine structure by an aliphatic radical comprising a fluorine atom is tolerated with respect to VMAT-2 binding in this series of novel fluoroalkyl tetrabenazine carbinol compounds enables the compounds of present invention to be used as positron emission tomography (PET) imaging agents in studies targeting the VMAT-2 biomarker.

Thus, in one embodiment, the present invention provides radiolabeled fluoroalkyl tetrabenazine carbinol compounds falling within the scope of generic structure I comprising a fluorine-18 atom. In an alternate embodiment, the present invention provides radiolabeled fluoroalkyl tetrabenazine carbinol compounds falling within the scope of generic structure IV comprising a fluorine-18 atom. Fluorine-18 labeled fluoroalkyl tetrabenazine carbinol compounds I and fluoroalkyl tetrabenazine carbinol compounds IV are suitable for use as imaging agents for positron emission tomography (PET) screening of human patients for example, for pathological conditions related to diabetes. Positron emission tomography has become a medical imaging technique of critical importance to human health.

In an alternate embodiment, the present invention provides fluoroalkyl tetrabenazine carbinol compounds falling within the scope of generic structure I and comprising a fluorine-19 atom, a stable isotope of fluorine. The fluoroalkyl tetrabenazine carbinol compounds comprising a fluorine-19 atom are useful in binding studies which allow the identification of those fluoroalkyl tetrabenazine carbinol compounds possessing optimal affinity for a target biomarker, for example VMAT-2. A substantial binding affinity of a given fluorine-19 containing fluoroalkyl tetrabenazine carbinol compound for a target biomarker such as VMAT-2 is a reliable predictor of utility in PET imaging of the corresponding fluorine-18 containing fluoroalkyl tetrabenazine carbinol compound. As is disclosed herein, fluoroalkyl tetrabenazine carbinol compounds I and IV show substantial binding affinity for the biomarker VMAT-2.

Although throughout this disclosure there is considerable focus on human health, the fluoroalkyl tetrabenazine carbinol compounds provided by the present invention are useful in the study and treatment of variety of human and animal diseases as imaging agents, as probes for the development of imaging agents, and as therapeutic agents.

Fluoroalkyl tetrabenazine carbinol compounds having structure I are illustrated in Table 1 below.

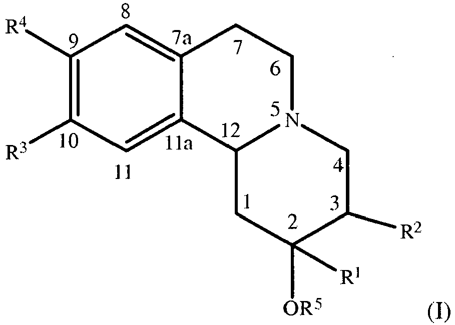

**Table 1 Examples Of Fluoroalkyl Tetrabenazine Carbinol Compounds Having Structure I**

| | | | | | | Ring Position* Stereochemistry | | |
|---|---|---|---|---|---|---|---|---|
| Entry | R¹ | R² | R³ | R⁴ | R⁵ | RP-2 | RP-3 | RP-12 |
| 1a | 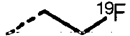 | 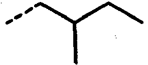 | CH₃ | CH₃ | Ac | R/S | R/S | R/S |
| 1b | 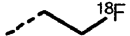 | 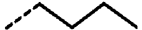 | CH₃ | CH₃ | Ac | R | R | R |
| 1c | 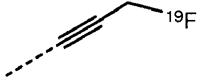 | 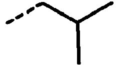 | CH₃O | CH₃O | Ph | R/S | R/S | R/S |
| 1d | 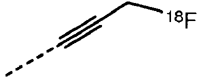 | 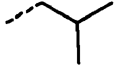 | CH₃O | CH₃O | H | S | S | S |
| 1e | 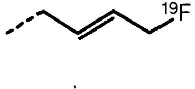 | 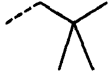 | EtO | CH₃O | Ph | R | S | R |
| 1f | 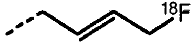 | 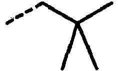 | EtO | EtO | Ac | S | R | S |
| 1g | 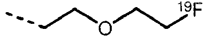 | 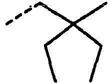 | CH₃CH₂ | CH₃ | Ph | R/S | R/S | R/S |
| 1h | 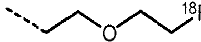 | 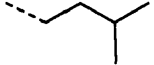 | CH₃O | CH₃O | Ac | R | R | R |
| 1i | 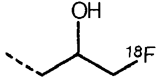 | 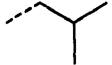 | CH₃O | CH₃O | H | R/S | R/S | R/S |
| 1j | 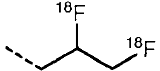 | 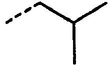 | CH₃O | CH₃ | Ac | R/S | R/S | R/S |
| 1k | 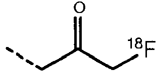 | 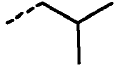 | CH₃O | H | H | R | R | R |

| | | | | | | | | |
|---|---|---|---|---|---|---|---|---|
| * RP-2 = Ring position-2, RP-3 = Ring position-3, RP-12 = Ring position-12 | | | | | | | | |

In general, and throughout this disclosure, where no absolute or relative stereochemistry is shown for a structure, as in for example structure I, the structure is intended to encompass all possible absolute and relative stereochemical configurations. Thus, structure I depicts a fluoroalkyl tetrabenazine carbinol compound in which no absolute or relative stereochemistry is shown. As such, structure I is intended to represent a genus of fluoroalkyl tetrabenazine carbinol compounds which includes the racemic compound 1a (Table 1) having both the R configuration and S configuration at ring positions-2, -3 and -12. In another embodiment, structure I represents fluoroalkyl tetrabenazine carbinol compound 1b (Table 1) having the R configuration (absolute stereochemistry) at ring positions-2, -3 and -12. In yet another embodiment, structure I represents compound 1d (Table 1) having absolute stereochemistry opposite that of compound 1b. Those having ordinary skill in the art will appreciate that the individual fluoroalkyl tetrabenazine carbinol compounds shown in Table 1 herein are illustrative of tetrabenazine (TBZ) derivatives falling within the scope of generic structure I.

As noted, in one embodiment, the present invention provides a fluoroalkyl tetrabenazine carbinol compound having structure I which may be a racemic mixture (e.g. compound 1a (Table 1), a single enantiomer (e.g. compound 1b (Table 1), or a composition enantiomerically enriched in a single principal component enantiomer. Entries 2a-2c in Table 2 below illustrate fluoroalkyl tetrabenazine carbinol compounds I comprising a principal component enantiomer and at least one minor component enantiomer.

**Table 2 Fluoroalkyl Tetrabenazine Carbinol Compounds I Comprising A Principal Component Enantiomer And At Least One Minor Component Enantiomer.**

| Entry | Structure of Principal Component Enantiomer | Structure of Minor Component Enantiomer |
|---|---|---|
| 2a | 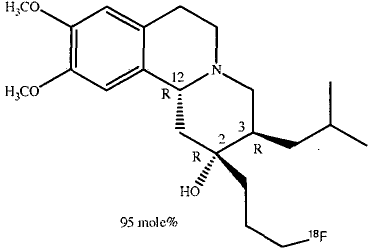 | 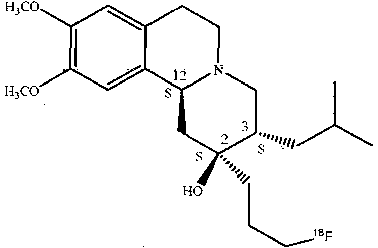 |
| 2b | 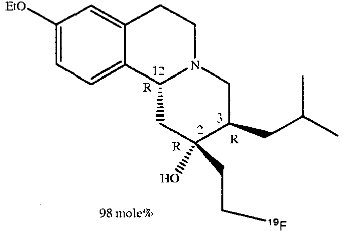 | 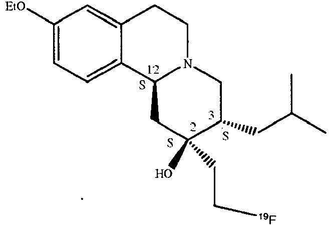 |
| 2c | 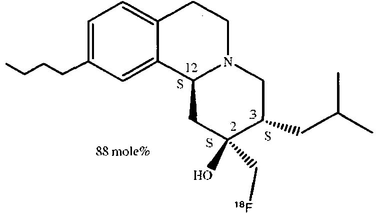 | 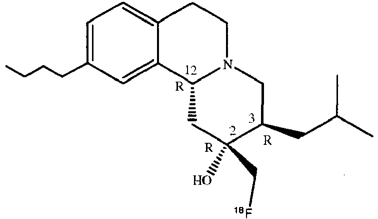 |

In Table 2 the fluoroalkyl tetrabenazine carbinol compositions comprise a principal component enantiomer (the structures appearing under the title heading "Structure of Principal Component Enantiomer") and a "Minor Component Enantiomer". In the fluoroalkyl tetrabenazine carbinol compositions illustrated in Table 2 the mole percentage of the principal component enantiomer is given as "mole%" and refers to the mole percentage of the principal component enantiomer having the structure shown relative to the amounts of all other fluoroalkyl tetrabenazine carbinol components in the composition. For the purposes of this discussion a fluoroalkyl tetrabenazine carbinol is any compound falling within the scope of generic structure I. Entry 2a represents a fluoroalkyl tetrabenazine carbinol composition comprising 95 mole% of the R, R, R principal component enantiomer shown and a lesser amount of the S, S, S minor component enantiomer. Entry 2c represents a fluoroalkyl tetrabenazine carbinol composition comprising 88 mole percent of the S, S, S principal component enantiomer having the structure shown and a lesser amount of the R, R, R minor component enantiomer. Those skilled in the art will appreciate that the fluoroalkyl tetrabenazine carbinol compositions provided by the present invention may comprise a principal component enantiomer, a minor component enantiomer, and additional diastereomeric fluoroalkyl tetrabenazine carbinol components. In one embodiment, the present invention provides a fluoroalkyl tetrabenazine carbinol composition comprising a principal component enantiomer and related diastereomers. In an alternate embodiment, the present invention provides a fluoroalkyl tetrabenazine carbinol composition having no principal component enantiomer and which is a diastereomeric mixture.

In one embodiment, the present invention provides a fluoroalkyl tetrabenazine carbinol compound represented by structure I, which is enantiomerically enriched and is comprised of at least 95 mole percent (mole %) of an enantiomer having the R configuration at ring position-12.

In an alternate embodiment, the present invention provides a fluoroalkyl tetrabenazine carbinol compound represented by structure I, which is enantiomerically enriched and is comprised of at least 95 mole percent (mole %) of an enantiomer having the R configuration at ring position-2,

In one embodiment, the present invention provides a fluoroalkyl tetrabenazine carbinol compound having structure I in which the fluorinated aliphatic radical at ring position-2 (-R¹) has a syn-configuration relative to the hydrogen at ring position-12. The principal component enantiomers of Entries 2a-2b of Table 2 illustrate fluoroalkyl tetrabenazine carbinol compounds in which the fluorinated aliphatic moiety at ring position-2 (-R¹) has a syn-configuration relative to the hydrogen at ring position-12.

In one embodiment, the present invention provides an enantiomerically enriched fluoroalkyl tetrabenazine carbinol compound comprising a principal component enantiomer having structure II 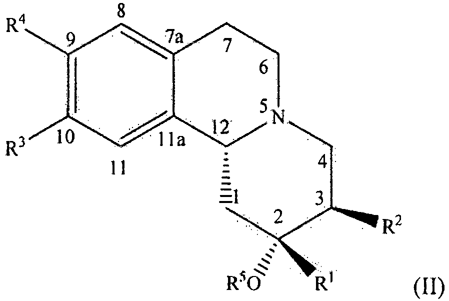 wherein R¹; R²; R³ ; R⁴ and R⁵ **are as defined for structure I.**

Principal component enantiomers having structure II are illustrated in Table 3 below.

**Table 3 Principal Component Enantiomers Having Structure II**

| Entry | Structure |
|---|---|
| 3a | 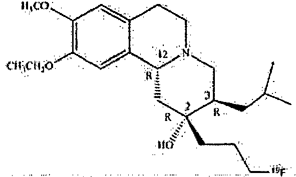 |
| 3b | 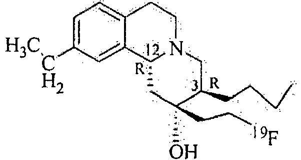 |
| 3c | 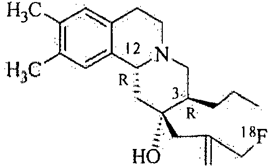 |

In one embodiment, the present invention provides an enantiomerically enriched fluoroalkyl tetrabenazine carbinol compound comprising at least 80 mole percent of an enantiomer having structure II, for example the composition comprising the compound of Entry 3a (Table 3) wherein the R, R, R enantiomer shown represents at least 80 mole percent relative to the amounts of all other fluoroalkyl tetrabenazine carbinol components in the composition.

In an alternate embodiment, the present invention provides an enantiomerically enriched fluoroalkyl tetrabenazine carbinol compound which is comprised of at least 95 mole % of an enantiomer having structure II, for example a fluoroalkyl tetrabenazine carbinol composition comprising the compound of Entry 3b (Table 3) wherein the enantiomer shown represents at least 95 mole percent relative to the amounts of all other fluoroalkyl tetrabenazine carbinol components in the composition.

Enantiomerically enriched fluoroalkyl tetrabenazine carbinol compounds outside the scope of the present invention comprise a principal component enantiomer having structure III 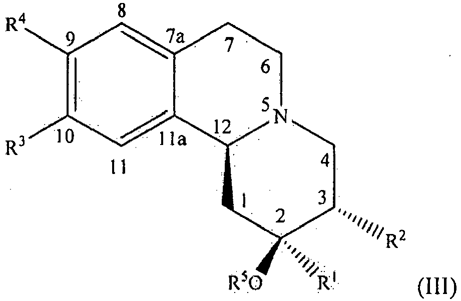 wherein R¹; R²; R³ ; R⁴ and R⁵ **are as defined for structure I.**

Principal component enantiomers having structure III are illustrated in Table 4 below.

**Table 4 Principal Component Enantiomers Having Structure III**

| Entry | Structure |
|---|---|
| 4a | 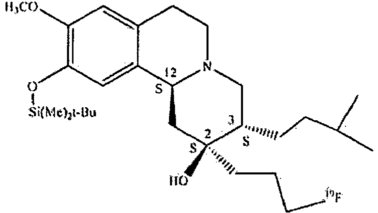 |
| 4b | 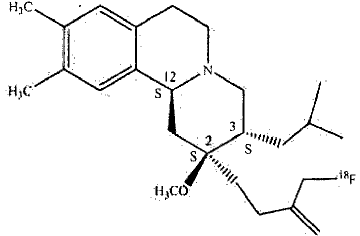 |
| 4c | 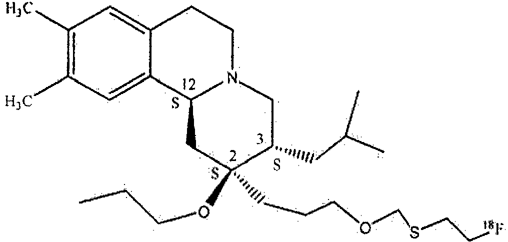 |

As noted, with respect to structures I, II, and III, in one embodiment, the group -OR⁵ is not a hydroxy group and is instead an ester moiety, for example an acetate group as exemplified by Entry 1a of Table 1, or for example an aryl ether moiety, for example a phenoxy group as exemplified by Entry 1g of Table 1. In one embodiment, the group -OR⁵ is an aliphatic ester moiety selected from the group consisting of acetate, propanoate, butanoate, pentanoate, hexanoate, and heptanoate.

As noted, the present invention provides novel fluoroalkyl tetrabenazine carbinol compounds I and IV, and in certain embodiments, mixtures thereof. Fluoroalkyl tetrabenazine carbinol compounds having structure IV are illustrated in Table 5 below.

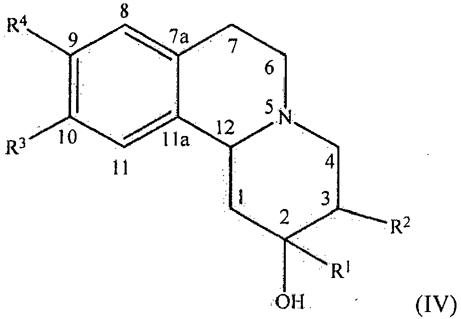

**Table 5 Examples Of Fluorophilic Tetrabenazine Carbinol Compound Having Structure IV**

| Entry | R¹ | R² | R³ | R⁴ | Ring Position* Stereochemistry | | |
|---|---|---|---|---|---|---|---|
| | | | | | RP-2 | RP-3 | RP-12 |
| 5a | 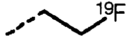 | 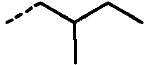 | CH₃ | CH₃ | R/S | R/S | R/S |
| 5b | 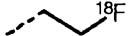 | 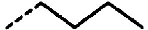 | CH₃ | CH₃ | R | R | R |
| 5c | 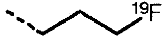 | 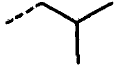 | CH₃O | CH₃O | R/S | R/S | R/S |
| 5d | 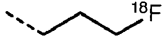 | 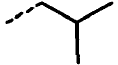 | CH₃O | CH₃O | S | S | S |
| 5e | 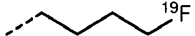 | 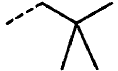 | EtO | CH₃O | R | R | R |
| 5f | 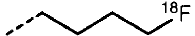 | 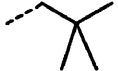 | EtO | EtO | S | S | S |
| 5g | 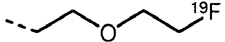 | 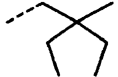 | CH₃CH₂ | CH₃CH₂ | R/S | R/S | R/S |
| 5h | 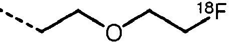 | 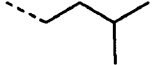 | CH₃O | CH₃O | R | R | R |
| 5i | 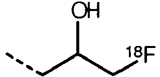 | 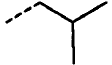 | CH₃O | CH₃O | R/S | R/S | R/S |
| 5j | 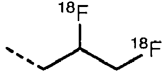 | 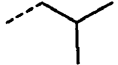 | CH₃O | CH₂CH₃ | R/S | R/S | R/S |
| 5k | 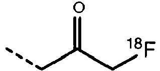 | 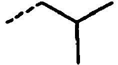 | CH₃O | H | R | R | R |

Structure IV represents a genus of fluoroalkyl tetrabenazine carbinol compounds which includes the racemic compound 5a (Table 5) having both the R configuration and S configuration at ring positions-2, -3, and -12. In another embodiment, structure IV represents fluoroalkyl tetrabenazine carbinol compound 5b (Table 5) having the R configuration (absolute stereochemistry) at ring positions-2, -3, and -12. In yet another embodiment, structure IV represents compound 5d (Table 5) having absolute stereochemistry opposite that of compound 5b. Those having ordinary skill in the art will appreciate that the individual fluoroalkyl tetrabenazine carbinol compounds shown in Table 5 herein are illustrative of tetrabenazine carbinol derivatives falling within the scope of generic structure IV. Those skilled in the art will appreciate as well that fluoroalkyl tetrabenazine carbinol compounds 5a, 5c, 5g, 5i and 5j represent racemic mixtures.

As noted, in one embodiment, the present invention provides a fluoroalkyl tetrabenazine carbinol compound having structure IV which may be a racemic mixture (e.g. compound 5a (Table 5), a single enantiomer (e.g. compound 5b (Table 5), or a composition enantiomerically enriched in a single principal component enantiomer. Entries 6a-6c in Table 6 below illustrate fluoroalkyl tetrabenazine carbinol compounds IV comprising a principal component enantiomer and at least one minor component enantiomer.

**Table 6 Fluoroalkyl Tetrabenazine Carbinol Compounds IV Comprising A Principal Component Enantiomer And At Least One Minor Component Enantiomer.**

| Entry | Structure of Principal Component Enantiomer | Structure of Minor Component Enantiomer |
|---|---|---|
| 6a | 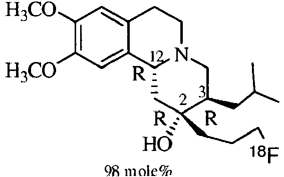 | 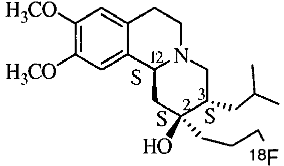 |
| 6b | 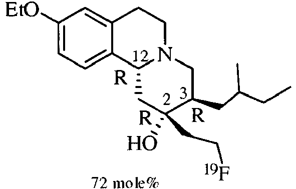 | 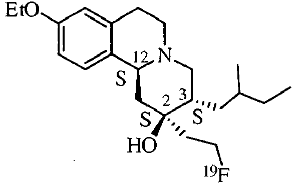 |
| 6c | 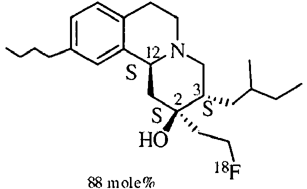 | 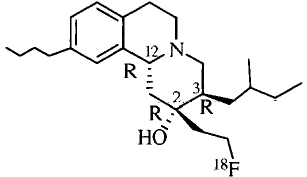 |

In Table 6 the fluoroalkyl tetrabenazine carbinol compositions comprise a principal component enantiomer and a minor component enantiomer. In the fluoroalkyl tetrabenazine carbinol compositions illustrated in Table 6 the mole percentage of the principal component enantiomer is given as "mole%" and refers to the mole percentage of the principal component enantiomer having the structure shown relative to the amounts of all other fluoroalkyl tetrabenazine carbinol components in the composition. For the purposes of this discussion a fluoroalkyl tetrabenazine carbinol is any compound falling within the scope of generic structure I. Those skilled in the art will appreciate that all compounds falling within the scope of generic structure IV also fall within the scope of generic structure I. Entry 6a represents a fluoroalkyl tetrabenazine carbinol composition comprising 98 mole% of the R, R, R principal component enantiomer shown and a lesser amount of the S, S, S minor component enantiomer. Entry 6c represents a fluoroalkyl tetrabenazine carbinol composition comprising 88 mole percent of the S, S, S principal component enantiomer having the structure shown and a lesser amount of the R, R, R minor component enantiomer.

In one embodiment, the present invention provides a fluoroalkyl tetrabenazine carbinol compound represented by structure IV, which is enantiomerically enriched and is comprised of at least 95 mole percent (mole %) of an enantiomer having the R configuration at ring position-12.

In an alternate embodiment, the present invention provides a fluoroalkyl tetrabenazine carbinol compound represented by structure IV, which is enantiomerically enriched and is comprised of at least 95 mole percent (mole %) of an enantiomer having the R configuration at ring position-2,

In one embodiment, the present invention provides a fluoroalkyl tetrabenazine carbinol compound having structure IV in which the fluorinated aliphatic radical at ring position-2 (-R¹) has a syn-configuration relative to the hydrogen at ring position-12. The principal component enantiomers of Entries 6a-6c of Table 6 illustrate fluoroalkyl tetrabenazine carbinol compounds in which the fluorinated aliphatic moiety at ring position-2 (-R¹) has a syn-configuration relative to the hydrogen at ring position-12.

In one embodiment, the present invention provides an enantiomerically enriched fluoroalkyl tetrabenazine carbinol compound comprising a principal component enantiomer having structure V 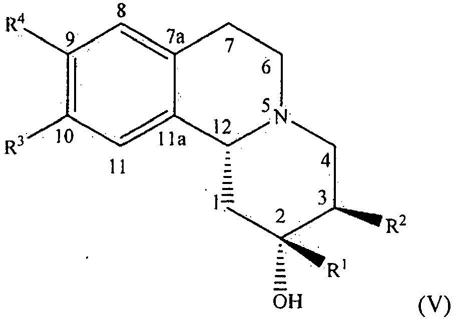 wherein R¹; R²; R³ ; and R⁴ are as defined for structure I.

Principal component enantiomers having structure V are illustrated in Table 7 below.

**Table 7 Principal Component Enantiomers Having Structure V**

| Entry | Structure |
|---|---|
| 7a | 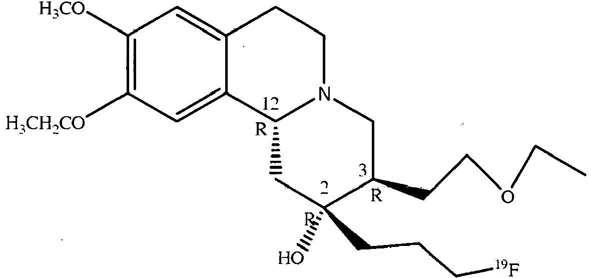 |
| 7b | 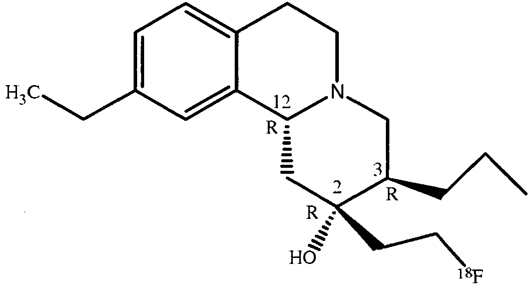 |
| 7c | 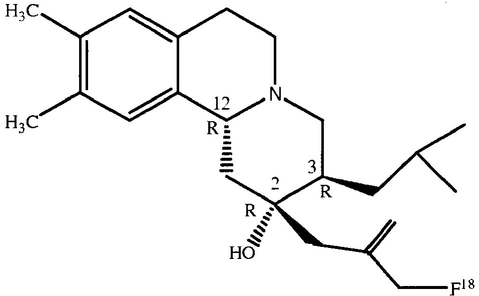 |

In one embodiment, the present invention provides an enantiomerically enriched fluoroalkyl tetrabenazine carbinol compound comprising at least 80 mole percent of an enantiomer having structure V, for example the composition comprising the compound of Entry 7a (Table 7) wherein the R, R, R enantiomer shown represents at least 80 mole percent relative to the amounts of all other fluoroalkyl tetrabenazine carbinol components in the composition.

In an alternate embodiment, the present invention provides an enantiomerically enriched fluoroalkyl tetrabenazine carbinol compound, which is comprised of at least 95 mole % of an enantiomer having structure V, for example an fluoroalkyl tetrabenazine carbinol composition comprising the compound of Entry 7b (Table 7 wherein the R, R, R enantiomer shown represents at least 95 mole percent relative to the amounts of all other fluoroalkyl tetrabenazine carbinol components in the composition.

In one embodiment, the present invention provides an enantiomerically enriched fluoroalkyl tetrabenazine carbinol compound comprising a principal component enantiomer having structure VI 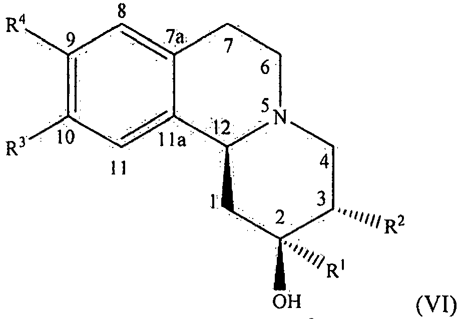 wherein R¹; R²; R³ and R⁴ are as defined for structure I.

Principal component enantiomers having structure VI are illustrated in Table 8 below.

**Table 8 Principal Component Enantiomers Having Structure VI**

| Entry | Structure |
|---|---|
| 8a | 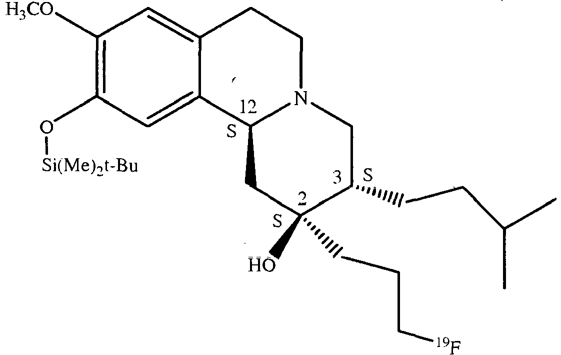 |
| 8b | 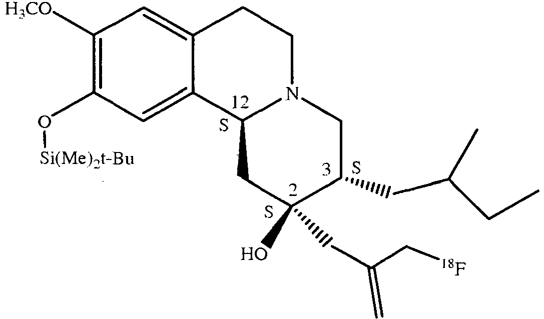 |
| 8c | 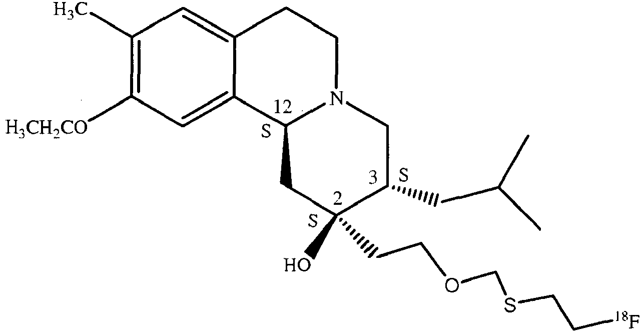 |

In one embodiment, the present invention provides an enantiomerically enriched fluoroalkyl tetrabenazine carbinol compound comprising at least 80 mole percent of an enantiomer having structure VI, for example a fluoroalkyl tetrabenazine carbinol composition comprising the compound of Entry 8a (Table 8) wherein the S, S, S enantiomer shown represents at least 80 mole percent relative to the amounts of all other fluoroalkyl tetrabenazine carbinol components in the composition. In another embodiment, the present invention provides an enantiomerically enriched fluoroalkyl tetrabenazine carbinol compound comprising at least 95 mole percent of an enantiomer having structure VI, for example a fluoroalkyl tetrabenazine carbinol composition comprising the compound of Entry 8b (Table 8) wherein the S, S, S enantiomer shown represents at least 95 mole percent relative to the amounts of all other fluoroalkyl tetrabenazine carbinol components in the composition.

In another embodiment, the present invention provides an enantiomerically enriched fluoroalkyl tetrabenazine carbinol compound having structure IV, wherein R¹ is a C₁-C₁₀ fluorinated aliphatic radical comprising at least one fluorine-18 atom; R² is a C₅-C₁₀ aliphatic radical; and R³ and R⁴ are methoxy groups.

As will be clear to one of ordinary skill in the art, the term "fluoroalkyl " refers to the group R¹ of structures I-VI which represents a C₁-C₁₀ aliphatic radical and is not restricted to the ordinary meaning of the term "alkyl". Thus although the term fluoroalkyl tetrabenazine carbinol is used extensively herein for convenience and means a tetrabenazine compound falling within the scope of generic structure I and comprising a C₁-C₁₀ fluorinated aliphatic radical at ring position-2.

As demonstrated below, the fluorine-18 labeled compounds of formula (I), (II), (III), (IV), (V), and (VI) have use as PET imaging agents for the VMAT-2 biomarker. Therefore, according to a further aspect of the invention, there is provided a method for detection of VMAT-2 in a subject, comprising :
(i) administration of a fluorine-18 labeled compound of formula (I), (II), (III), (IV), (V) or (VI) as defined above, or a salt thereof to said subject;
(ii) detecting uptake of said fluorine-18 labeled compound by *in vivo* PET imaging.

Such a method provides information and data having utility in the diagnosis and clinical research of VMAT-2 related disorders, for example by providing a method for determining beta cell mass. The subject is a mammal, most suitably a human who has or is suspected of having a VMAT-2 related disorder. In an alternative aspect, a compound of formula (I), (II), (III), (IV), (V) or (VI) as defined above, or a salt thereof may also be used to image VMAT-2 in healthy human volunteers for example for clinical research purposes. The imaging of VMAT-2 may be carried out quantitatively such that the amount or change in amount of VMAT-2 may be determined so as to diagnose or track progress of a disease. Alternatively the imaging of VMAT-2 may be used to locate VMAT-2.

The term "VMAT-2 related disorder" means a VMAT-2 related disease in the brain such as Huntington's, Parkinson's, or schizophrenia, or of the pancreas such as beta cell associated disorder including an insulinoma or other neuroendocrine tumour, or diabetes (for example type 1 diabetes, type 2 diabetes, or preclinical type 1 diabetes). In one aspect of the invention, the VMAT-2 related disorder is diabetes. In an alternative aspect of the invention, the VMAT-2 related disorder is schizophrenia.

Accordingly, there is further provided use of a fluorine-18 labeled compound of formula (I), (II), (III), (IV), (V) or (VI) or a salt thereof in the manufacture of a radiopharmaceutical for the *in vivo* PET diagnosis or imaging of a VMAT-2 related disorder. In the alternative, there is provided a fluorine-18 labeled compound of formula (I), (II), (III), (IV), (V) or (VI) or a salt thereof for use in the *in vivo* PET diagnosis or imaging of a VMAT-2 related disorder.

In a further aspect, there is provided a method for the *in vivo* diagnosis or imaging of a VMAT-2 related disorder in a subject, preferably a human, comprising administration of a fluorine-18 labeled compound of formula (I), (II), (III), (IV), (V) or (VI) or a salt thereof and detecting the uptake of said compound by an *in vivo* PET imaging technique. The method is especially preferred for the *in vivo* diagnosis or imaging of diabetes. In one aspect of the invention, the method comprises detecting the uptake of a fluorine-18 labeled compound of formula (I), (II), (III), (IV), (V) or (VI) or a salt thereof by an *in vivo* PET imaging technique in a subject, preferably a human to whom said compound has been pre-administered.

The invention further provides a method of monitoring the effect of treatment of a subject, preferably a human with a drug to combat a VMAT-2 related disorder, said method comprising administering to said subject a fluorine-18 labeled compound of formula (I), (II), (III), (IV), (V) or (VI) or a salt thereof and detecting the uptake of said compound by an *in vivo* PET imaging technique, said administration and detection optionally but preferably being effected repeatedly, e.g. before, during and after treatment with said drug.

A compound of formula (I), (II), (III), (IV), (V) or (VI) or a salt thereof is preferably administered for *in vivo* use in a pharmaceutical formulation comprising said compound and a pharmaceutically acceptable excipient, such formulations thus form a further aspect of the invention. A "pharmaceutical formulation" is defined in the present invention as a formulation comprising an effective amount of a compound of formula (I), (II), (III), (IV), (V) or (VI) or a salt thereof in a form suitable for administration to a mammal, suitably a human. The "pharmaceutically acceptable excipient" is a suitably a fluid, especially a liquid, in which the compound of formula (I), (II), (III), (IV), (V) or (VI) or a salt thereof can be suspended or dissolved, such that the formulation is physiologically tolerable, ie. can be administered to the mammalian body without toxicity or undue discomfort. The pharmaceutically acceptable excipient is suitably an injectable carrier liquid such as sterile, pyrogen-free water for injection; an aqueous solution such as saline (which may advantageously be balanced so that the final formulation for injection is isotonic); an aqueous solution of one or more tonicity-adjusting substances (for example, salts of plasma cations with biocompatible counterions), sugars (for example, glucose or sucrose), sugar alcohols (for example, sorbitol or mannitol), glycols (for example. glycerol), or other non-ionic polyol materials (for example, polyethyleneglycols, propylene glycols and the like). Preferably the pharmaceutically acceptable excipient is pyrogen-free water for injection or isotonic saline.

The pharmaceutical formulation may optionally contain additional excipients such as an antimicrobial preservative, pH-adjusting agent, filler, stabiliser or osmolality adjusting agent. By the term "antimicrobial preservative" is meant an agent which inhibits the growth of potentially harmful micro-organisms such as bacteria, yeasts or moulds. The antimicrobial preservative may also exhibit some bactericidal properties, depending on the dosage employed. The main role of the antimicrobial preservative(s) of the present invention is to inhibit the growth of any such microorganism in the pharmaceutical formulation. The antimicrobial preservative may, however, also optionally be used to inhibit the growth of potentially harmful micro-organisms in one or more components of kits used to prepare said pharmaceutical formulation prior to administration. Suitable antimicrobial preservative(s) include: the parabens, ie. methyl, ethyl, propyl or butyl paraben or mixtures thereof; benzyl alcohol; phenol; cresol; cetrimide and thiomersal. Preferred antimicrobial preservative(s) are the parabens.

The term "pH-adjusting agent" means a compound or mixture of compounds useful to ensure that the pH of the pharmaceutical formulation is within acceptable limits (approximately pH 4.0 to 10.5) for human or mammalian administration. Suitable such pH-adjusting agents include pharmaceutically acceptable buffers, such as tricine, phosphate or TRIS [ie. *tris*(hydroxymethyl)aminomethane], and pharmaceutically acceptable bases such as sodium carbonate, sodium bicarbonate or mixtures thereof. When the pharamceutical formulation is employed in kit form, the pH adjusting agent may optionally be provided in a separate vial or container, so that the user of the kit can adjust the pH as part of a multi-step procedure.

By the term "filler" is meant a pharmaceutically acceptable bulking agent which may facilitate material handling during production and lyophilisation. Suitable fillers include inorganic salts such as sodium chloride, and water soluble sugars or sugar alcohols such as sucrose, maltose, mannitol or trehalose.

The pharmaceutical formulations of the invention are typically supplied in suitable vials or vessels which comprise a sealed container which permits maintenance of sterile integrity and/or radioactive safety, plus optionally an inert headspace gas (eg. nitrogen or argon), whilst permitting addition and withdrawal of solutions by syringe or cannula. A preferred such container is a septum-sealed vial, wherein the gas-tight closure is crimped on with an overseal (typically of aluminium). The closure is suitable for single or multiple puncturing with a hypodermic needle (e.g. a crimped-on septum seal closure) whilst maintaining sterile integrity. Such containers have the additional advantage that the closure can withstand vacuum if desired (eg. to change the headspace gas or degas solutions), and withstand pressure changes such as reductions in pressure without permitting ingress of external atmospheric gases, such as oxygen or water vapour.

Preferred multiple dose containers comprise a single bulk vial (e.g. of 10 to 30 cm³ volume) which contains multiple patient doses, whereby single patient doses can thus be withdrawn into clinical grade syringes at various time intervals during the viable lifetime of the preparation to suit the clinical situation. Pre-filled syringes are designed to contain a single human dose, or "unit dose" and are therefore preferably a disposable or other syringe suitable for clinical use. The pharmaceutical formulations of the present invention preferably have a dosage suitable for a single patient and are provided in a suitable syringe or container, as described above.

The pharmaceutical formulations of the invention may be prepared under aseptic manufacture (ie. clean room) conditions to give the desired sterile, non-pyrogenic product. It is preferred that the key components, especially the excipients plus those parts of the apparatus which come into contact with the pharmaceutical formulation (for example, vials) are sterile. The components of the pharmaceutical formulation can be sterilised by methods known in the art, including: sterile filtration, terminal sterilisation using, for example, gamma-irradiation, autoclaving, dry heat or chemical treatment (for example, with ethylene oxide). It is preferred to sterilise some components in advance, so that the minimum number of manipulations needs to be carried out. As a precaution, however, it is preferred to include at least a sterile filtration step as the final step in the preparation of the pharmaceutical formulation.

An "effective amount" of a compound of formula (I), (II), (III), (IV), (V) or (VI) or a salt thereof means an amount which is effective for use in *in vivo* PET imaging or for use in therapy and will vary depending on the exact compound to be administered, the weight of the subject or patient, and other variables as would be apparent to a physician skilled in the art. The fluorine-18 labeled compounds of this invention may be administered to a subject for PET imaging in amounts sufficient to yield the desired signal, typical radionuclide dosages of 0.01 to 100 mCi, preferably 0.1 to 50 mCi will normally be sufficient per 70kg bodyweight.

As noted, the fluoroalkyl tetrabenazine carbinol compounds I, II, III, IV, V, and VI provided by the present invention may comprise a fluorine-18 atom in the fluorinated aliphatic moiety -R¹. In various embodiments such fluoroalkyl tetrabenazine carbinol compounds comprising a fluorine-18 atom are useful as PET imaging agents. Thus, in one embodiment, the present invention provides a PET imaging agent comprising a fluoroalkyl tetrabenazine carbinol compound having structure I 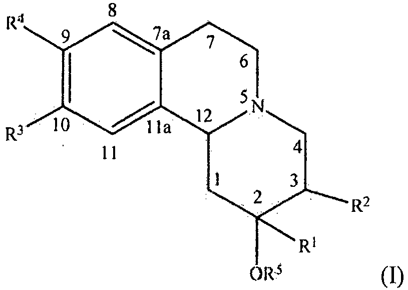 wherein R¹ is as defined above and comprises at least one fluorine-18 atom; R², R³, R⁴ and R⁵ are as defined above.

In another embodiment, the present invention provides a PET imaging agent comprising an enantiomerically enriched fluoroalkyl tetrabenazine carbinol compound comprising a principal component enantiomer having structure II 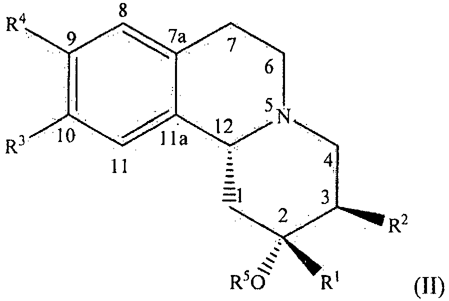 wherein R¹ is as defined above and comprises at least one fluorine-18 atom; R², R³, R⁴ and R⁵ are as defined above.

In yet another embodiment, the present invention provides a PET imaging agent comprising a fluoroalkyl tetrabenazine carbinol compound having structure IV 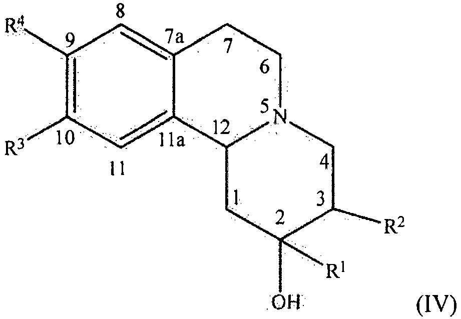 wherein R¹ is as defined above and comprises at least one fluorine-18 atom; R²; R³; and R⁴ are as defined for structure I.

In another embodiment, the present invention provides a PET imaging agent comprising an enantiomerically enriched fluoroalkyl tetrabenazine carbinol compound comprising a principal component enantiomer having structure V 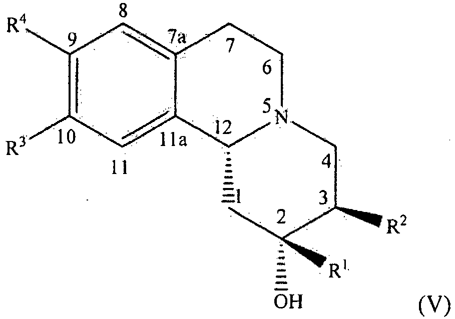 wherein R¹ is as defined above and comprises at least one fluorine-18 atom; R²; R³; and R⁴ are as defined for structure I.

The term "PET imaging agent" as used herein refers to a composition comprising a fluorine-18 labeled fluoroalkyl tetrabenazine carbinol compound, which may be administered to a patient in order to perform a PET scan. Typically, the imaging agent is presented to the patient in the form of an aqueous formulation containing a sufficient amount of fluorine-18 labeled fluoroalkyl tetrabenazine carbinol compound to conduct the PET scan. Typically, the amount of fluorine-18 labeled fluoroalkyl tetrabenazine carbinol compound presented to a patient corresponds to a weight of the fluorine-18 labeled fluoroalkyl tetrabenazine carbinol compound on the order of nanograms. In reference to the relative amounts of non-radioactive fluorine-19 containing fluoroalkyl tetrabenazine carbinol compound present in the PET imaging agent presented to a patient, the PET imaging agent typically has a specific activity in a range from about 1 to about 99 percent. In one embodiment, the PET imaging agent has a specific activity in a range from about 10 to about 95 percent. In another embodiment, the PET imaging agent has a specific activity in a range from about 20 to about 90 percent.

The aqueous formulation containing the fluorine-18 fluoroalkyl tetrabenazine carbinol compound is typically administered intravenously and may contain various agents, which promote the dispersal of the PET imaging agent in water. In one embodiment, the PET imaging agent may be administered to a patient in an aqueous formulation comprising ethanol and the fluorine-18 labeled fluoroalkyl tetrabenazine carbinol compound. In an alternate embodiment, the PET imaging agent may be administered to a patient as an aqueous formulation comprising dextrose and the fluorine-18 labeled fluoroalkyl tetrabenazine carbinol compound. In yet another embodiment, the PET imaging agent may be administered to a patient as an aqueous formulation comprising saline and the fluorine-18 labeled fluoroalkyl tetrabenazine carbinol compound. Such a formulation may optionally contain further excipients as described above, more typically including one or more excipients such as buffers; pharmaceutically acceptable solubilisers (e.g. cyclodextrins or surfactants such as Pluronic, Tween or phospholipids); pharmaceutically acceptable stabilisers or antioxidants (such as ascorbic acid, gentisic acid or *para*-aminobenzoic acid.

In addition to being useful as PET imaging agents and as probes for determining the suitability of a given fluoroalkyl tetrabenazine carbinol compound for use as a PET imaging agent, the fluoroalkyl tetrabenazine carbinol compounds provided by the present invention are believed to possess therapeutic utility in the treatment of diseases such as schizophrenia and Huntinaton's disease. Thus, in one embodiment, the present invention provides a fluoroalkyl tetrabenazine carbinol compound having structure I, which is useful in treating a pathological condition in a patient. In an alternate embodiment, the present invention provides a fluoroalkyl tetrabenazine carbinol compound having structure IV, which is useful in treating a pathological condition in a patient. In various other embodiments, the present invention provides enantiomerically enriched fluoroalkyl tetrabenazine carbinol compounds II, III, V, and VI (and mixtures thereof), which are useful in treating a pathological condition in a patient. Typically the amount of amount of the fluoroalkyl tetrabenazine carbinol compound administered to a patient in a given dose is on the order of milligrams.

Those skilled in the art will appreciate that fluoroalkyl tetrabenazine carbinol compounds falling within the scope of generic structure I may under a variety of conditions form salts which are useful as PET imaging agents, probes for the discovery and development of imaging agents, and/or as therapeutic agents. Thus, the present invention provides a host of novel and useful fluoroalkyl tetrabenazine carbinol compounds and their salts.

Suitable salts according to the invention include (i) physiologically acceptable acid addition salts such as those derived from mineral acids, for example hydrochloric, hydrobromic, phosphoric, metaphosphoric, nitric and sulphuric acids, and those derived from organic acids, for example tartaric, trifluoroacetic, citric, malic, lactic, fumaric, benzoic, glycollic, gluconic, succinic, methanesulphonic, and paratoluenesulphonic acids; and (ii) physiologically acceptable base salts such as ammonium salts, alkali metal salts (for example those of sodium and potassium), alkaline earth metal salts (for example those of calcium and magnesium), salts with organic bases such as triethanolamine, N-methyl-D-glucamine, piperidine, pyridine, piperazine, and morpholine, and salts with amino acids such as arginine and lysine. In one particular embodiment, the present invention provides the hydrochloride salts of the novel fluoroalkyl tetrabenazine carbinol compounds, for example the hydrochloride salt of the compound of Entry 4a of Table 4.

In a further aspect of the invention, there is provided a compound of formula (I), (II), (III), (IV), (V), or (VI) or a salt thereof, for use in medicine.

The fluoroalkyl tetrabenazine carbinol compounds of the present invention may be prepared by a variety of methods including those provided in the experimental section of this disclosure. In one embodiment, the fluoroalkyl tetrabenazine carbinol compound is prepared by reaction of nucleophilic fluoride ion or an electrophilic fluorinating agent with a fluorophilic tetrabenazine carbinol compound having structure VII 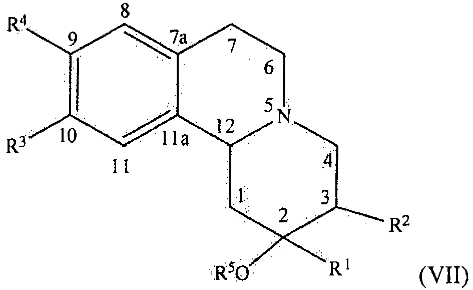 wherein R¹ is a C₁-C₂₀ aliphatic radical comprising at least one functional group susceptible to reaction with nucleophilic fluoride ion or an electrophilic fluorinating agent; R²; R³; R⁴; and R⁵ are as defined for structure I.

Thus in one embodiment, the present invention provides a fluorophilic tetrabenazine carbinol compound having structure VII. Fluorophilic tetrabenazine carbinol compounds having structure VII are illustrated in Table 9 below.

**Table 9 Examples Of Fluorophilic Tetrabenazine Compounds Having Structure VII**

| Entry | R¹ | R² | R³ | R⁴ | R⁵ | Ring Position* Stereochemistry | | |
|---|---|---|---|---|---|---|---|---|
| | | | | | | RP-2 | RP-3 | RP-12 |
| 9a | 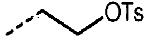 | 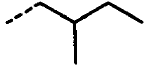 | CH₃ | CH₃ | Ac | R/S | R/S | R/S |
| 9b | 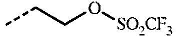 | 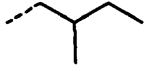 | CH₃ | CH₃ | Ac | R | R | R |
| 9c | 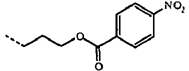 | 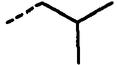 | CH₃O | CH₃O | Ph | R/S | R/S | R/S |
| 9d | 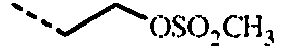 | 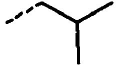 | CH₃O | CH₃O | H | S | S | S |
| 9e | 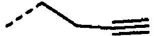 | 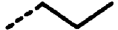 | EtO | CH₃O | Ph | R | S | R |
| 9f | 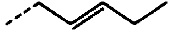 | 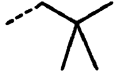 | EtO | EtO | Ac | S | R | S |
| 9g | 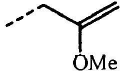 | 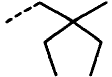 | CH₃CH₂ | CH₃CH₂ | Ph | R/S | R/S | R/S |
| 9h | 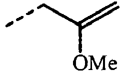 | 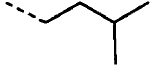 | CH₃O | CH₃O | Ac | R | R | R |
| 9i | 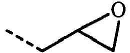 | 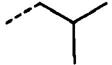 | CH₃O | CH₂CH₃ | H | R/S | R/S | R/S |
| 9j | 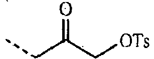 | 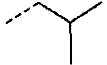 | CH₃O | H | Ac | R/S | R/S | R/S |
| 9k | 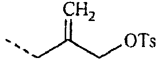 | 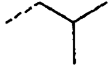 | CH₃O | CH₃O | H | R | R | R |
| 9l | 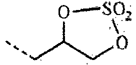 | 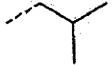 | CH₃O | CH₃O | Ph | R | R | R |

As noted, in one embodiment, the present invention provides a fluorophilic compound having structure VII, wherein R¹ is a C₁-C₂₀ aliphatic radical, comprising at least one functional group susceptible to reaction with nucleophilic fluoride ion. In one embodiment, the functional group susceptible to reaction with nucleophilic fluoride ion is an aromatic sulfonate ester (e.g. tosylate, benzenesulfonate, naphthalenesulfonate). In an alternate embodiment, the functional group susceptible to reaction with nucleophilic fluoride ion is an aliphatic sulfonate ester (e.g. methane sulfonate, trifluoromethane sulfonate). In one embodiment, the functional group susceptible to reaction with nucleophilic fluoride ion is selected from the group consisting of tosylate, mesylate, and trifluoromethane sulfonate groups.

In one embodiment, the present invention provides a fluorophilic compound having structure VII wherein the group R¹ comprises at least one tosylate group susceptible to reaction with nucleophilic fluoride ion. See for example the Entries 9a, 9j and 9k of Table 9. As defined herein, the tosylate group is an aromatic radical and the group R¹ comprising the tosylate group is also an aromatic radical. In the compound shown in Entry 9a for example, the group R¹ comprising the tosylate group is a C₉ aromatic radical which upon displacement with fluoride ion becomes a C₂ fluorinated aliphatic radical.

In an alternate embodiment, the present invention provides a fluorophilic compound having structure VII wherein the group R¹ comprises at least one mesylate group susceptible to reaction with nucleophilic fluoride ion.

In an alternate embodiment, the present invention provides a fluorophilic compound having structure VII wherein the group R¹ comprises at least one trifluoromethane sulfonate (triflate) group susceptible to reaction with nucleophilic fluoride ion. See for example Entry 9b of Table 9.

In an alternate embodiment, the present invention provides a fluorophilic compound having structure VII wherein the group R¹ comprises at least one p-nitrobenzoate group susceptible to reaction with nucleophilic fluoride ion. See for example Entry 9c of Table 9.

In an alternate embodiment, the present invention provides a fluorophilic compound having structure VII wherein the group R¹ comprises at least one methane sulfonate group susceptible to reaction with nucleophilic fluoride ion. See for example Entry 9d of Table 9.

In an alternate embodiment, the present invention provides a fluorophilic compound having structure VII wherein the group R¹ comprises at least one epoxy group susceptible to reaction with nucleophilic fluoride ion. See for example Entry 9i of Table 9.

In yet another embodiment, the present invention provides a fluorophilic compound having structure VII wherein the group R¹ comprises at least one cyclic sulfate group susceptible to reaction with nucleophilic fluoride ion. See for example Entry 91 of Table 9.

In one embodiment, the present invention provides a fluorophilic compound having structure VII, wherein R¹ is a C₂-C₂₀ aliphatic radical comprising at least one functional group susceptible to reaction with an electrophilic fluorinating agent, for example fluorine gas, perchloryl fluoride, mercuric fluoride, and phenyl selenenyl fluoride.

Thus in one embodiment, the functional group susceptible to reaction with an electrophilic fluorinating agent is selected from the group consisting of carbon-carbon double bonds and carbon-carbon triple bonds. Entries 9e, 9f, 9g, 9h and 9k of Table 9 illustrate compounds falling within the scope of generic structure VII, which are susceptible to reaction with an electrophilic fluorinating agent. Attention is called to Entry 9k wherein the group R¹ comprises functional groups susceptible to reaction with an electrophilic fluorinating agent (double bond) and to reaction with nucleophilic fluoride ion (tosylate group).

Fluorophilic tetrabenazine carbinol compounds VII may be prepared in enantiomerically enriched or racemic forms. For example, a fluorophilic tetrabenazine compound VII may be enriched in the R, R, R- enantiomer shown in Entry 9b of Table 9. Alternatively, a fluorophilic tetrabenazine carbinol compound may be enriched in an enantiomer having absolute stereochemistry opposite that of Entry 9b of Table 9, for example the S, S, S-enantiomer of Entry 9d.

Thus, in one embodiment, the present invention provides an enantiomerically enriched fluorophilic tetrabenazine carbinol compound comprising a principal component enantiomer having structure VIII 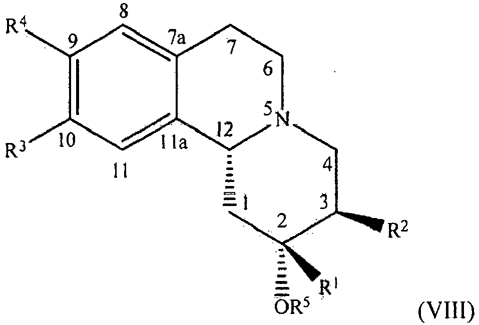 wherein R¹ is a C₁-C₁₀ aliphatic radical comprising at least one functional group susceptible to reaction with nucleophilic fluoride ion or an electrophilic fluorinating agent; R²; R³; R⁴; and R⁵ are as defined for structure I. Principal component enantiomers VIII are illustrated by Entries 9b, 9h, 9k, and 9l of Table 9. aliphatic radical; and R⁵ is hydrogen, a C₁-C₁₀ aliphatic radical, a C₂-C₁₀ cycloaliphatic radical, or a C₂-C₂₀ aromatic radical. Principal component enantiomers IX are illustrated by Entry 9d of Table 9.

As noted, with respect to structures VII, VIII, and XI, in one embodiment, the group-OR⁵ is not a hydroxy group and is instead a C₁-C₁₀ aliphatic radical, a C₂-C₁₀ cycloaliphatic radical, or a C₂-C₂₀ aromatic radical. Thus in one embodiment, the group -OR⁵ is an ester moiety, for example an acetate group as exemplified by Entry 9a of Table 9, or for example an aryl ether moiety, for example a phenoxy group as exemplified by Entry 9c of Table 9. In one embodiment, the group -OR⁵ is an aliphatic ester moiety selected from the group consisting of formate, acetate, propanoate, butanoate, pentanoate, hexanoate, and heptanoate. In an alternate embodiment, the group -OR⁵ is a silyl ether moiety, for example triethylsilyloxy.

Co-pending International Patent Application PCT/US3008/065738 discloses methods for the preparation of racemic and enantiomerically enriched tetrabenazine compositions which may be used for the preparation of fluorophilic tetrabenazine carbinol compounds provided by the present invention. In addition, the Examples Section of the present disclosure provides detailed experimental descriptions of the preparation and characterization of tetrabenazine carbinol compounds VII.

In general, tetrabenazine carbinol compounds VII can be prepared from the corresponding tetrabenazine compound. Tetrabenazine compounds may be prepared by reacting a nucleophilic alkenyl species with an aldehyde compound having structure X 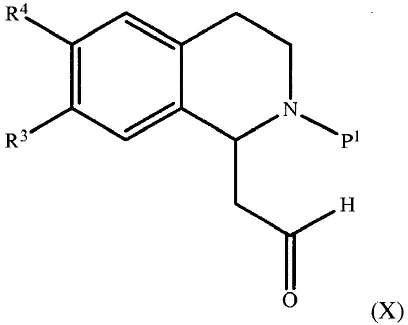 wherein R³ and R⁴ are as defined for structure I; and P¹ is a protecting group, to provide an allylic alcohol (See Method 4 of the Examples section), which is then oxidized to provide an enone designated the "first intermediate" (See Example 1 of the Examples section), the protecting group of which is then removed and the resultant deprotected first intermediate undergoes an amino cyclization reaction to afford the corresponding tetrabenazine (TBZ) compound.

Representative aldehyde compounds encompassed by generic formula X are given in Table 10.

**Table 10 Representative Aldehyde Compounds Encompassed By Formula X**

| Entry | Compound Type | Ring Position* Stereochemistry | Structure |
|---|---|---|---|
| 10a | Single "R" enantiomer. "Boc" protecting group P¹ | RP-12 "R" | 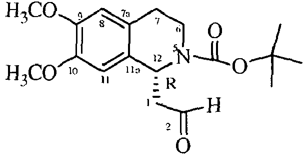 |
| 10b | Single "S" enantiomer. "Boc" protecting group P¹ | RP-12 "S" | 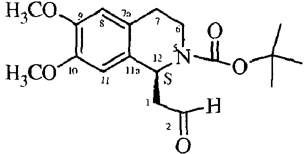 |
| 10c | Enantiomeric ally enriched mixture of "R" and "S" enantiomers, "alloc" protecting group P¹ | RP-12 "R/S" | 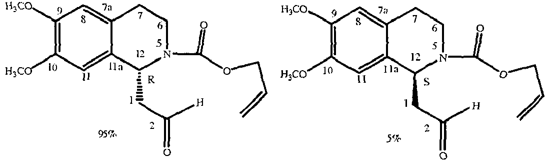 |
| 10d | Racemic mixture of "R" and "S" enantiomers; "Fmoc" protecting group P¹ | RP-12 "R/S" | 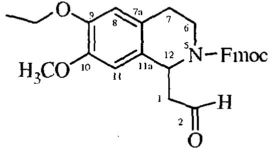 |
| 10e | Racemic mixture of "R" and "S" enantiomers; "Cbz" protecting group P¹ | RP-12 "R/S" | 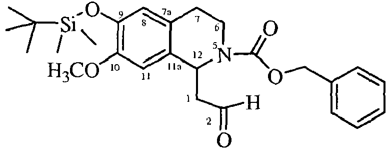 |
| 10f | Racemic mixture of "R" and "S" enantiomers; "Teoc" protecting group P¹ | RP-12 "R/S" | 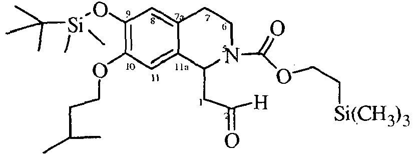 |
| 10g | Single "R" enantiomer. "Boc" protecting group P¹ | RP-12 "R" | 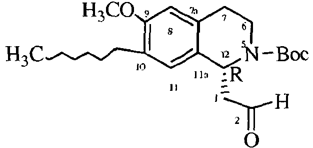 |

The preparation of the aldehyde compound featured in Entry 10a of Table 10 is described in the Examples section of this disclosure (Methods 1-3). In general, the class of aldehyde compounds represented by structure X may be prepared by art recognized methods, for example using the methodology depicted in Scheme 1.

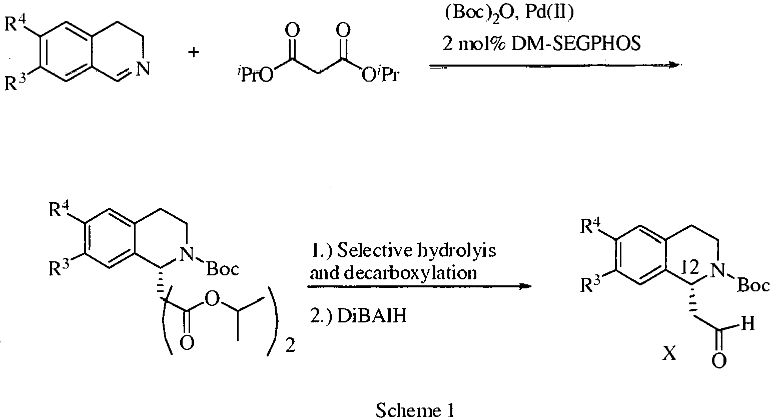

Thus, aldehyde compounds X may be prepared from intermediates prepared using methodology described by Sasamoto et al. (Journal of the American Chemical Society 128, 14010-14011, 2006). Sasamoto et al. disclose the preparation of enantiomerically enriched tetrahydroquinoline malonate compounds, which may be converted to aldehyde compound X by selective hydrolysis of one of the ester moieties of the tetrahydroquinoline malonate and decarboxylation followed by reduction of the resultant tetrahydroisoquinoline monoester to aldehyde compound X as depicted in Scheme I.

One of ordinary skill in the art will appreciate that the 2 mole percent DM-SEGPHOS shown is Scheme 1 represents a chiral catalyst responsible for the enantiomeric enrichment of the product aldehyde X, and further that the use of DM-SEGPHOS of opposite chirality as the chiral catalyst will afford a product aldehyde X enantiomerically enriched in the "S" enantiomer (aldehyde compound X having the S configuration at ring position-12 (See for example Entry 10b of Table 10). Suitable chiral catalysts include those disclosed by Sasamoto et al. (Journal of the American Chemical Society 128, 14010-14011, 2006) for example (S)-Binap, (R)-Binap, (S)-DM-Binap, (R)-DM-Binap, (S)-DM-SEGPHOS, and (R)-DM-SEGPHOS. Typically use of a catalyst consisting of a ligand possessing a single, for example "S", configuration produces stereochemically enriched malonate adducts of the opposite "R" configuration and *vice versa.*

In addition to the use of a chiral catalyst to generate aldehyde compounds X enriched in a single configuration at ring position-12, there are available a wide variety of methods for the separation of racemic aldehyde X into its constituent enantiomers. For example, racemic aldehyde compound X may be separated into its constituent enantiomers by high performance liquid chromatography (hplc) on a chiral hplc column.

Other methods for producing enantiomerically enriched compositions provided by the present invention include conversion of a racemic fluoroalkyl tetrabenazine carbinol compound having structure I into an adduct comprising a mixture of diastereomers which are then separated by fractional crystallization. For example, a racemic fluoroalkyl tetrabenazine carbinol compound having structure I may be reacted with (-)-tartaric acid to form an adduct (ammonium tartarate salt) of the racemic fluoroalkyl tetrabenazine carbinol compound, said adduct comprising a mixture of diastereomeric ammonium tartarate salts which are then separated by fractional crystallization.

### EXAMPLES

### Method 1 Preparation of Protected Diester 2

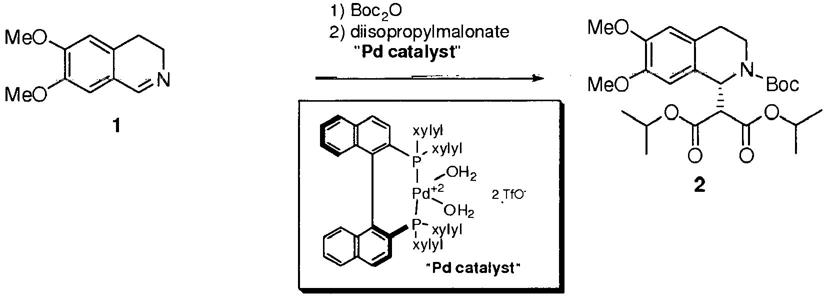

The dihydroisoquinoline 1 (1.0 eq.) and Boc anhydride (1.5 eq.) were dissolved in CH₂Cl₂ at room temperature to provide a 1.5 M solution with respect to the dihydroisoquinoline. The mixture was allowed to stir for 30 min. Following the allotted time, the reaction mixture was cooled to 0 °C and then diisopropylmalonate (1.5 eq.) followed by a pre-chilled solution of the Pd catalyst (0.008 eq.) in dichloromethane were added successively to the reaction mixture to provide a final reaction concentration of 0.84 M with respect to the starting dihydroisoquinoline. The reaction mixture was allowed to continue stirring at ∼2.5 °C for 15 h. Following this time EtOAc and brine were added to the reaction mixture. The aqueous layer was extracted with three portions of EtOAc and the combined organic layers were dried (Na₂SO₄), filtered, and concentrated under reduced pressure to provide the crude product. The crude material was dissolved in a minimal amount of dichloromethane and purified by flash chromatography on SiO₂ (15-30% EtOAc-hexanes, elution was observed at 285 nm and 228 nm). The product 2 was a colorless solid that existed as a mixture of rotamers in solution at room temperature 94%: [α]²⁶_{D} -69.0 ( c 0.21, CHCl₃); ¹H NMR (CDCl₃) δ 0.81-1.02 (m, 6H), 1.06-1.17 (m, 6H), 1.23-1.38 (m, 9H), 2.51-2.63 (m, 1H), 2.64-2.77 (m, 1H), 3.20-3.29 (m, 0.6H), 3.32-3.41 (m, 0.4H), 3.51-3.58 (m, 1H), 3.62-3.70 (m, 6H), 3.70-3.76 (m, 0.4H), 3.91-4.01 (m, 0.6H), 4.65-4.82 (m, 1H), 4.83-4.98 (m, 1H), 5.71 (apparent d, J = 5.7 Hz, 0.6H), 5.78 (apparent d, *J* = 7.9 Hz, 0.4H), 6.42-6.49 (m, 1H), 6.77 (s, 0.6H), 6.81 (s, 0.4H); ¹³C NMR (CDCl₃) δ 21.02, 21.09, 21.18, 21.32, 27.24, 27.95, 28.02, 37.60, 39.34, 52.11, 52.83, 55.48, 55.52, 59.28, 60.08, 68.58, 68.76, 68.82, 79.46, 80.03, 110.09, 110.73, 111.13, 126.11, 126.18, 126.37, 127.07, 146.81, 146.87, 147.93, 153.86, 154.30, 166.29, 166.78, 166.94, 167.06.

### Method 2 Selective Hydrolysis and Decarboxylation of Protected Ester 3

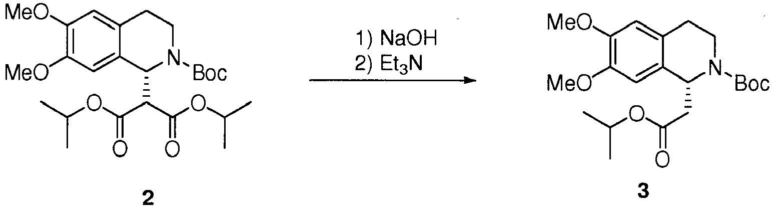

The starting material 2 was taken up in isopropanol to provide a 0.2 M solution of 2. To this solution was added 1M aqueous NaOH, bringing the final concentration of the reaction mixture to 0.1M with respect to the malonate **2.** The reaction mixture was heated to and maintained 70 °C for 22 min. (timing was started when the temperature of the reaction mixture temp exceeded 65 °C). Following the allotted time the reaction mixture was quickly cooled to 0 °C. The reaction mixture carefully acidified with 2M aqueous HCl and extracted with three portions of dichloromethane. The combined organic extracts dried (Na₂SO₄), filtered and concentrated under reduced pressure. The isolated material was taken up in THF to provide a 0.1 M solution (based on the original quantity of **2** used in the reaction mixture) and triethylamine (1.0 eq) was added to the reaction mixture at room temperature. The reaction mixture was heated to its reflux temperature and maintained at this temperature for 90 min. The reaction mixture was concentrated under reduced pressure, dissolved in a minimal quantity of CH₂Cl₂ and was immediately purified by column chromatography on SiO₂ (15-40% EtOAc-hexanes; 40%, the eluant was monitored at 284 nm). The product **3** existed as a mixture of rotamers at room temperature and was a colorless foam 79%: [α]²⁶_{D} -82 ( c 0.24, CH₂Cl₂); ¹H NMR (CDCl₃) δ 1.19-1.25 (m, 6H), 1.43-1.49 (m, 9H), 2.58-2.69 (m, 2H), 2.70-2.77 (m, 1H), 2.78-2.92 (m, 1H), 3.13-3.43 (m, 1H), 3.81-3.85 (m, 6H), 3.86-4.01 (m, 1H), 4.91-5.05 (m, 1H), 5.38-5.61 (m, 1H), 6.56-6.61 (m, 1H), 6.64-6.70 (s, 1H); ¹³C NMR (CDCl₃) δ 21.75, 21.90, 27.93, 28.08, 28.44, 37.53, 38.75, 42.22, 42.81, 51.11, 51.87, 55.92, 56.02, 68.08, 79.74, 80.21, 109.60, 109.99, 111.44, 111.54, 126.28, 126.48, 128.54, 128.76, 147.51, 147.97, 154.39, 154.51, 170.36, 170.59; LRMS-(ESI+) calcd for (C₂₁H₃₁NO₆ + H) ([M+H]⁺ 394.22, found 394.16.

### Method 3 Preparation of Aldehyde Compound 4

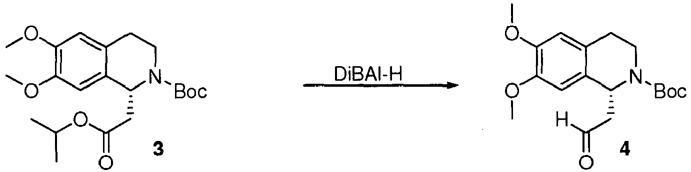

To a 0.12 M solution of the starting monoester **(3,** 1.0 eq.) in toluene at -78 °C was added a 1.5 M solution of DiBAl-H in hexanes (1.5 eq.) dropwise via a syringe pump. Following the addition the reaction mixture was stirred at -78 °C for 2 h. The reaction mixture was quenched by the addition of EtOAc and was then acidified with saturated aqueous citric acid solution. The reaction mixture was allowed to warm to room temperature and continue stirring for 30 min. The phases were separated, and the aqueous layer extracted with three portions of EtOAc. The combined organic extracts were washed with two portions of 2 M aqueous HCl solution, brine, dried (MgSO₄), filtered, and concentrated under reduced pressure. The crude product was subjected purification on SiO₂ (15-35% EtOAc-hexanes; Elution was observed at 285 nm and 228 nm). The isolated product, aldehyde compound **4,** was a colorless foam. The product existed as a 1:1 mixture of rotamers at room temperature 76%: [α]²⁶_{D} -116 ( c 0.26,CH₂Cl₂); ¹H NMR (CDCl₃) δ 1.40 (s, 9H), 2.58 (apparent t, *J*= 3.8 Hz, 0.5H), 2.61 (apparent t, *J* = 3.5 Hz, 0.5H), 2.68-2.88 (m, 3H), 3.02-3.27 (m, 1H), 3.78 (apparent s, 6H), 3.87-3.99 (m, 0.5H), 4.08-4.23 (m, 0.5H), 5.37-5.68 (m, 1H), 6.55 (s, 1H), 6.58 (s, 1H), 9.78 (s, 1H); ¹³C NMR (CDCl₃) δ 20.90, 28.02, 28.27, 37.23, 38.65, 49.29, 49.93, 51.12, 55.83, 55.96, 80.13, 80.64, 109.42, 109.52, 111.52, 126.34, 126.51, 127.78, 127.82, 147.72, 147.97, 153.85, 154.62, 200.08, 200.33.

### Method 4 Reaction Of Aldehyde Compound 4 With Nucleophilic Alkenyl Species Derived From Alkenyl Iodide 5 With To Provide Allylic Alcohol 6

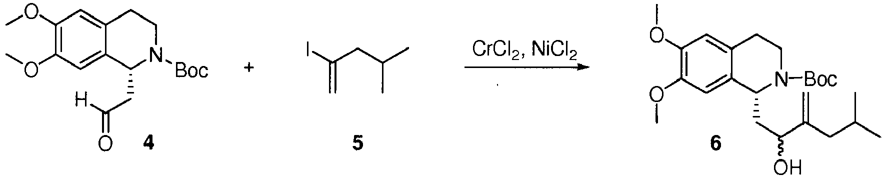

To a neat mixture of the alkenyl iodide 5 (1.0 eq) and the aldehyde compound **4** (1.0 eq.) at room temperature was added 2.65 eq. of chromium chloride doped with 0.5% NiCl₂ (w/w). The mixture was vortexed for about 2 min. to provide a homogeneous, green/grey paste and then stirred under nitrogen for an additional 10 min. after which time anhydrous DMF was added to bring the final reaction concentration to 0.36 M. The reaction mixture was deep green in color and was permitted to continue stirring at room temperature for 14h. Following the allotted time, the reaction mixture was diluted with 1:1 EtOAc-hexanes and an aqueous 0.5 M EDTA solution (pH 9) was added and the entire mixture was allowed to stir for 1.5 h. The aqueous layer was extracted with three portions of EtOAc, dried (MgSO₄), filtered, and the filtrate was concentrated under reduced pressure to provide a green oil. The crude material was subjected to column chromatography on SiO₂ (35% EtOAc-hexanes; elution was observed at 285 nm and 228 nm). The product allylic alcohol **6** was a pale yellow oil isolated in 53% yield as a mixture of diastereomers, which was taken on to the next step without additional characterization or analysis.

### Method 5 Oxidation Of Allylic Alcohol 6 To Provide First Intermediate 8

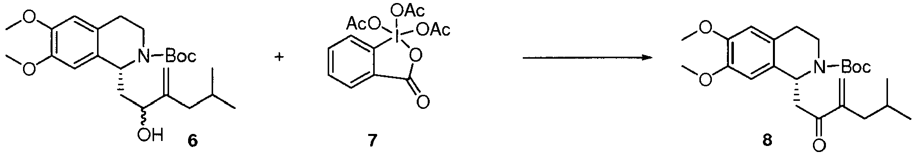

To a 0.1 M solution of allylic alcohol **6** (1.0 eq) in dichloromethane at 0 °C was added 1.1 eq. of the Dess-Martin reagent **7.** The reaction mixture was allowed to stir, slowly warming to room temperature over 2.5 h. The reaction was quenched by the addition of saturated aqueous sodium bicarbonate solution and diluted with ethyl acetate. The organic and aqueous layers were partitioned and separated and the aqueous layer extracted with three additional portions of ethyl acetate. The combined organic extracts were washed with brine, dried (MgSO₄), filtered, and concentrated under reduced pressure. The crude material was purified by column chromatography on SiO₂ (10-30% EtOAc-hexanes, elution was observed at 285 nm and 228 nm). The product first intermediate **8** was a colorless, foul-smelling oil that existed at 26 °C as a 60:40 mixture of rotamers in solution (66%): ¹H NMR (CDCl₃) δ 0.82 (apparent t, *J*= 7.6 Hz, 6H), 1.42 (s, 9H), 1.70 (apparent sept, *J* = 6.62 Hz, 1H), 2.08-2.15 (m, 1H), 2.15-2.24 (m, 1H), 2.62-2.70 (m, 1H), 2.75-2.91 (m, 1H), 2.93-3.07 (m, 1H), 3.07-3.29 (m, 1.6H), 3.30-3.43 (m, 0.4H), 3.79 (s, 3H), 3.81 (s, 3.4H), 4.04-4.16 (m, 0.6H), 5.52-5.62 (m, 1H), 5.69 (s, 1H), 5.90 (s, 0.6H), 6.04 (s, 0.4H), 6.57 (s, 1H), 6.63 (s, 1H); ¹³C NMR (CDCl₃) δ 22.45, 27.04, 27.25, 28.11, 28.41, 38.01, 39.33, 40.39, 45.20, 45.90, 51.62, 55.92, 55.98, 79.75, 80.23, 109.85, 110.25, 110.28, 111.41, 125.65, 125.72, 126.26, 129.25, 147.57, 147.87, 148.16, 148.29, 148.35, 154.40, 154.51, 199.53; HRMS-(ESI+) calcd for (C₂₄H₃₅NO₅) + H) ([M+H]⁺ 418.2594, found 418.2590.

### Method 5 Removal The Boc Protecting Group First Intermediate 8 And Amino Cyclization Provide (+)-Tetrabenazine 9

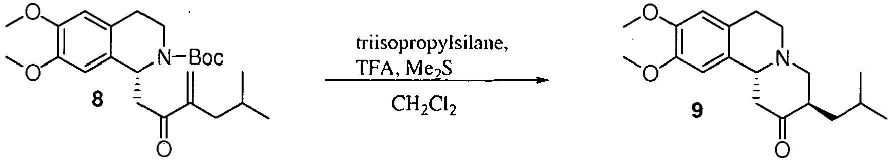

First intermediate **8** (1.0 eq) was dissolved in 10% Me₂S-dichloromethane to provide an 82 mM solution. The solution was cooled to 0 °C and triisopropylsilane (1.1 eq.) followed by TFA (precooled to 0 °C) was added to the reaction mixture to provide a final concentration of 41 mM. The reaction mixture was permitted to stir at 0 °C for 1 h. Following the allotted time the reaction mixture was quenched at 0 °C by the addition of saturated aqueous potassium carbonate solution and concentrated under reduced pressure to remove the majority of the dimethylsulfide. The mixture was extracted with five portions of dichloromethane, and the combined organic extracts were washed with brine, dried (MgSO₄), filtered and concentrated under reduced pressure to provide the crude product as a yellow solid. The crude product was recrystallized from 3.5% dimethoxyethane in hexanes. The resulting colorless crystals were washed with hexanes to provide pure (+)-tetrabenazine (9) 46%: mp 126.0 °C (3.5% DME-hexanes) (a crystal polymorph was observed at 116 °C); [α]²⁶_{D} +37.2 (c 0.41, CH₂Cl₂); ¹H NMR (CD₂Cl₂) δ 0.89 (apparent t, *J* = 7.2 Hz, 6H), 0.98 (ddd, *J =* 12, 6.0, 4.0 Hz, 1H), 1.59-1.68 (m, 1H), 1.74 (ddd, *J* = 12, 5.9, 5.7 Hz, 1H), 2.32 (apparent t, *J* = 11.7 Hz, 1H), 2.46 (apparent t, *J* = 12.3 Hz, 1H), 2.55 (ddd, *J* = 12, 10.0, 3.8 Hz, 1H), 2.65-2.73 (m, 2H), 2.83 (dd, *J* = 5.5, 2.8Hz, 1H), 2.97-3.07 (m, 1H), 3.07-3.14 (m, 1H), 3.25 (dd, *J* =9.7, 6.3 Hz, 1H), 3.47 (apparent d, *J* = 12Hz, 1H), 3.75 (s, 3H), 3.77 (s, 3H), 6.55 (s, 1H), 6.60 (s, 1H) ¹³C NMR (CD₂Cl₂) δ 21.98, 23.02, 25.51, 29.46, 35.16, 47.47, 47.63, 50.47, 55.87, 56.01, 61.47, 62.46, 108.46, 111.72, 126.37, 128.96, 147.65, 147.98, 209.72; HRMS-(ESI+) calcd for (C₁₉H₂₇NO₃ + H) ([M+H]⁺ 318.2069, found 318.2082.

### Example 1 Conversion of (+)-Tetrabenazine 9 into Tetrabenazine Carbinol Compound 12

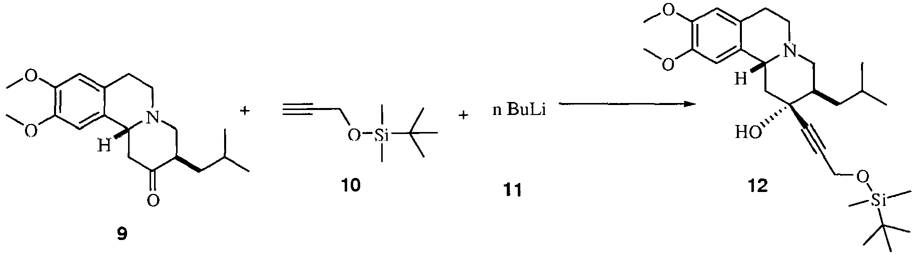

To *tert*-butyldimethyl(prop-2-ynaloxy)silane **10** (0.27 mL, 1.323 mmol) in THF (4 mL) was added nBuLi **11** (0.53 mL, 2.5 M in hexane, 1.323 mmol) dropwise. The mixture was stirred at 0 °C for 0.5 h. To the above reaction mixture tetrabenazine **9** (210 mg, 0.660 mmol) in THF (4 mL) was added dropwise over a period of 10 min. The reaction mixture was stirred at 0 °C for 1h. Following the allotted time the reaction mixture was quenched by the addition of saturated ammonium chloride (NH₄Cl). The mixture was extracted with three portions of EtOAc, and the combined organic extracts were washed with brine, dried over anhydrous Na₂SO₄, filtered and concentrated provide the crude product. The crude product was chromatographed on SiO₂ (12g, 10% to 60% EtOAc in hexane) to provide 210 mg of the product **12** (diastereomeric mixture, dr = 4:1) as a yellow solid (65%). ¹H NMR (CDCl₃) δ 6.64 (s, 1H), 6.54 (s, 1H), 4.40 (s, 2H), 3.84 (s, 6H), 3.46 (d, *J* = 10.0 Hz, 1H), 3.06-3.13 (m, 1H), 2.99-3.02 (m, 1H), 2.95 (dd, *J* = 15.0 & 5.0 Hz, 1H), 2.65 (d, *J* = 15.0 Hz, 1H), 2.49-2.58 (m, 3H), 2.28 (t, *J* = 10.0 Hz, 1H), 1.90-1.96 (m, 1H), 1.77 (t, *J* = 10.0 Hz, 1H), 1.63-1.70 (m, 1H), 1.49-1.54 (m, 1H), 1.21-1.27 (m, 1H), 0.95 (d, *J* = 6.6 Hz, 3H), 0.93 (d, *J* = 6.6 Hz, 3H), 0.91 (s, 9 H), 0.14 (s, 6H); ¹³C NMR (CDCl₃) δ 147.41, 147.10, 129.03, 126.48, 111.47, 107.82, 86.25, 85.11, 72.18, 59.71, 58.54, 55.91, 55.82, 51.76, 51.48, 45.57, 43.92, 37.17, 29.15, 25.80, 25.58, 24.10, 21.84, 18.27.

### Example 2 Esterification of Tetrabenazine Carbinol Compound 12 to Provide Acetate 13

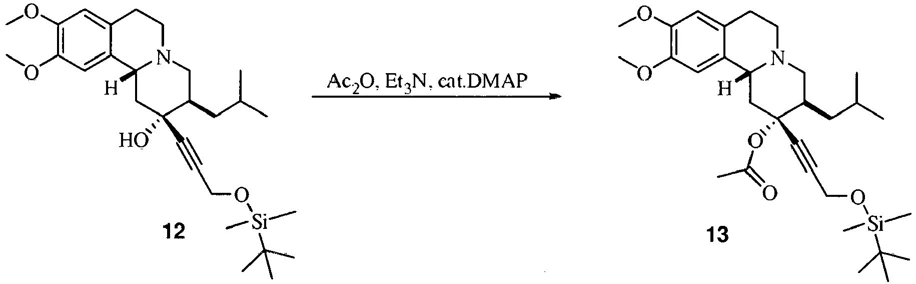

To a solution of (2*R*, 3*R*, 11b*R*)-2-(3-(tert-Butyldimethylsilyloxy)prop-1-ynyl)-3-isobutyl-9, 10-dimethoxy-2,3,4,6,7,11b-hexahydro-1-*H*-pyrido[2,1-*a*]isoquinolin-2-ol **12** (140 mg, 0.287 mmol) in CH₂Cl₂ (1 mL) (precooled to 0 °C) was added Ac₂O (60 µL, 0.631 mmol), Et₃N (0.12 mL, 0.861 mmol and DMAP (4 mg, 0.03 mmol). The reaction mixture was stirred for 14 h (0 °C to RT). Following the allotted time the reaction mixture was quenched with saturated sodium bicarbonate (NaHCO₃). The mixture was extracted with three portions of CH₂Cl₂ and the combined organic extracts were washed with brine, dried over anhydrous Na₂SO₄, filtered and concentrated to provide the crude product. The crude product was chromatographed on SiO₂ (12 g, 10% to 40% EtOAc in hexane) to provide 98 mg of the compound **13** (single diastereomer) as a slightly yellow solid (66%). ¹H NMR (CDCl₃) δ 6.66 (s, 1H), 6.57 (s, 1H), 4.42 (s, 2H), 3.85 (s, 3H), 3.83 (s, 3H), 3.50 (d, *J* = 11.00 Hz, 1H), 3.39 (dd, *J* = 12.47 & 2.16 Hz, 1H), 3.03-3.10 (m, 1H), 2.96-2.99 (m, 1H), 2.92 (dd, *J* = 12.17 & 4.04 Hz, 1H), 2.65 (d, *J* = 16.05 Hz, 1H), 2.53 (td, *J* = 11.43 & 4.08 Hz, 1H), 2.44 (t, *J* = 11.97 Hz, 1H), 2.12-2.16 (m, 1H), 2.04 (s, 3H), 1.65-1.70 (m, 2H), 1.49-1.55 (m, 1H), 1.20-1.25 (m, 1H), 0.95 (d, *J* = 6.6 Hz, 3H), 0.93 (d, *J* = 6.6 Hz, 3H), 0.91 (s, 9 H), 0.14 (s, 6H); ¹³C NMR (CDCl₃) δ 169.76, 147.89, 147.61, 129.31, 126.96, 111.88, 108.56, 88.11, 81.61, 79.27, 59.50, 58.31, 56.52, 56.19, 52.16, 51.79, 42.78, 41.59, 37.80, 29.61, 26.15, 26.09, 24.28, 22.44, 18.59, 4.61, 4.71.

### Example 3 Deprotection of Acetate 13 to Tetrabenazine Carbinol Compound 14

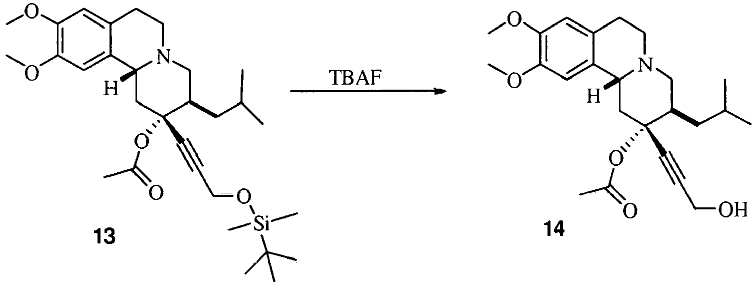

A solution of (2*R*, 3*R*, 11b*R*)-2-(3-(tert-Butyldimethylsilyloxy)prop-1-ynyl)-3-isobutyl-9,10-dimethoxy-2,3,4,6,7,11b-hexahydro-1-*H*-pyrido[2,1-a]isoquinolin-2-yl acetate **13** (98 mg, 0.185 mmol) in THF (1 mL) was added dropwise a solution of TBAF ( 0.56 mL, 1M in THF, 0.555 mmol) at 0 °C. The reaction mixture was stirred (0 °C to RT) for 2 h. After the allotted time the reaction mixture was quenched with saturated ammonium chloride (NH₄Cl). The mixture was extracted with three portions of EtOAc and the combined organic extracts were washed with brine, dried over anhydrous Na₂SO₄, filtered and concentrated to provide the crude product. The crude product was chromatographed on SiO₂ (12 g, 10% to 50% EtOAc in hexane) to produce 68 mg of the compound **14** as a slightly yellow solid (88%). ¹H NMR (CDCl₃) δ 6.67 (s, 1H), 6.57 (s, 1H), 4.35 (s, 2H), 3.86 (s, 3H), 3.83 (s, 3H), 3.49 (d, *J* = 12.00 Hz, 1H), 3.35 (d, *J* = 11.24 Hz, 1H), 3.04-3.10 (m, 1H), 2.97-3.00 (m, 1H), 2.92 (dd, *J* = 12.04 & 3.68 Hz, 1H), 2.75 (br s, 1H), 2.65 (d, *J* = 14.99 Hz, 1H), 2.49-2.54 (m, 1H), 2.39-2.44 (m, 1H), 2.10-2.16 (m, 1H), 2.05 (s, 3H), 1.63-1.70 (m, 2H), 1.50 (td, *J* = 13.58 & 2.95 Hz, 1H), 1.16-1.21 (m, 1H), 0.95 (d, *J* = 6.60 Hz, 3H), 0.93 (d, *J* = 6.60 Hz, 3H); ¹³C NMR (CDCl₃) δ 170.20, 147.99, 147.64, 129.03, 126.86, 111.92, 108.65, 88.42, 82.15, 79.31, 59.40, 58.22, 56.58, 56.20, 51.62, 51.31, 42.59, 41.49, 37.62, 29.49, 26.11, 26.05, 24.49, 22.42.

### Example 4 Preparation of Fluorophilic Tetrabenazine Carbinol Compound, Mesylate 15

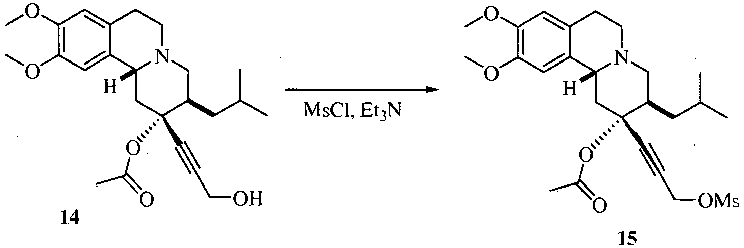

To a 0 °C solution of (2*R*, 3*R*, 11b*R*)-2-(3-hydroxyprop-1-ynyl)-3-isobutyl-9,10-dimethoxy-2,3,4,6,7,11b-hexahydro-1-*H*-pytido[2,1-*a*]isoquinolin-2-yl acetate **14** (60 mg, 0.144 mmol) in CH₂Cl₂ (1.5 mL) was added dropwise Et₃N (61 µL, 0.433 mmol) followed by methanesulfonyl chloride (MsCl) (17 µl, 0.216 mmol). The reaction mixture was stirred at 0 °C for about 2 h. After the allotted time the reaction mixture was poured into cold water and layers separated. The reaction mixture was extracted with three portions of CH₂Cl₂ and the combined organic extracts were dried over anhydrous Na₂SO₄, filtered and concentrated. The residue was chromatographed on SiO₂ (12 g, 10% to 70% EtOAc in hexane) to give 38 mg of the product as a yellow solid of compound **15** (53%).

### Example 5 Preparation of Fluoroalkyl Tetrabenazine Carbinol Compound 16

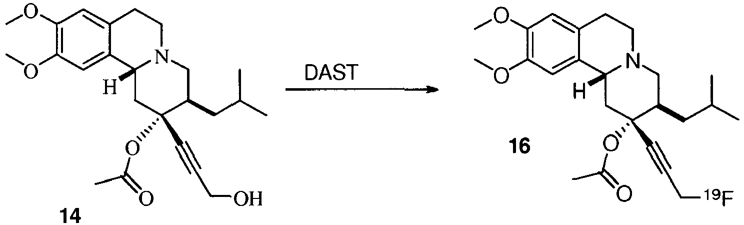

A solution of (2*R*, 3*R*, 11b*R*)-2-(3-hydroxyprop-1-ynyl)-3-isobutyl-9,10-dimethoxy-2,3,4,6,7,11b-hexahydro-1-*H*-pyrido[2,1-*a*]isoquinolin-2-yl acetate **14** (10 mg, 0.024 mmol) in CH₂Cl₂ (0.5 mL) was cooled to -78 °C. To this cooled solution was added dropwise a solution of (diethylamino)sulfur trifluoride (DAST) (10 µL, 0.072 mmol) in CH₂Cl₂ (0.1 mL). The reaction mixture was stirred (-78 °C to RT) for 4 h. The reaction mixture was then quenched with water and extracted with three portions of CH₂Cl₂. The combined organic extracts were dried over anhydrous Na₂SO₄, filtered and concentrated. The residue was chromatographed on SiO₂ (0% to 10% MeOH in CH₂Cl₂) to give 3 mg of the product 16 as a white solid (30%). ¹H NMR (CDCl₃) δ 6.68 (s, 1H), 6.60 (s, 1H), 5.10 (d, *J* = 47.43 Hz, 2H), 3.88 (s, 3H), 3.86 (s, 3H), 3.49 (d, *J* = 11.56 Hz, 1H), 3.40 (dd, *J* = 12.40 & 2.36 Hz, 1H), 3.06-3.12 (m, 1H), 2.95-3.02 (m, 2H), 2.68 (d, *J* = 15.12 Hz, 1H), 2.57 (td, *J* = 11.03 & 4.09 Hz, 1H), 2.44 (d, *J*= 11.85 Hz, 1H), 2.10-2.21 (m, 1H), 2.09 (s, 3H), 1.70 (t, *J*= 11.04, 2H), 1.51-1.56 (m, 1H), 1.20-1.25 (m, 1H), 0.99 (d, *J*= 6.62 Hz, 3H), 0.95 (d, *J*= 6.62 Hz, 3H).

### Example 6 Preparation of Fluoroalkyl Tetrabenazine Carbinol Compound 17

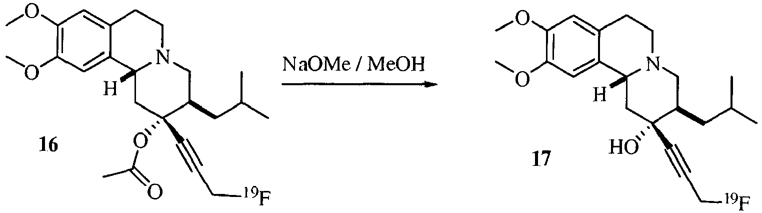

The starting fluoroalkyl tetrabenazine carbinol compound (2*R*, 3*R*, 11b*R*)-2-(3-fluoroprop-1-ynyl)-3-isobutyl-9,10-dimethoxy-2,3,4,6,7,11b-hexahydro-1-*H-*pyrido[2,1-*a*]isoquinolin-2-yl acetate **16** (1.8 mg, 0.0043 mmol) was added to 1 mL of a solution prepared by dissolution of freshly cut sodium (1 mg, 0.043 mmol) in cold MeOH (10 mL). The mixture was stirred at room temperature for 2 h. The mixture was concentrated under a stream of N₂ and dissolved in saturated ammonium chloride (NH₄Cl). Following the allotted time the mixture was extracted with three portions of CH₂Cl₂ and the combined organic extracts were dried over anhydrous Na₂SO₄, filtered, concentrated and dried under vacuum to give 1.5 mg of the product as a white solid **17** which was > 95% pure by HPLC analysis (92%). HRMS calcd. for (M+H) 376.2288, found 376.2290.

### Example 7 Preparation of Tetrabenazine Carbinol Compound, Diol 18

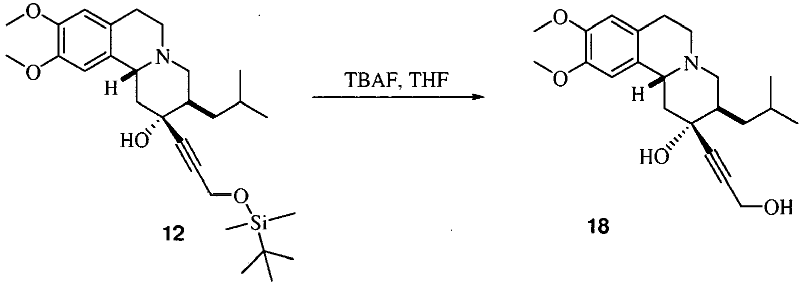

To a precooled (0 °C) solution of tetrabenazine carbinol compound, **12** (112 mg, 0.220 mmol) in 2 mL of THF, was added dropwise a solution of TBAF (330 ml, 0.33 mmol, 1M in THF). The mixture is stirred at room temperature for 12 h. Following the allotted time the reaction mixture was quenched by the addition of saturated ammonium chloride (NH₄Cl). The mixture was extracted with three portions of EtOAc, and the combined organic extracts were washed with brine, dried over anhydrous Na₂SO₄, filtered and concentrated to provide a product. The product was chromatographed on SiO₂ (12 g, 0% to 10% MeOH in CH₂Cl₂) to produce 49 mg of the tetrabenazine carbinol compound, diol **18** as a slightly yellow solid (60%). ¹H NMR (CDCl₃) δ 6.67 (s, 1H), 6.57 (s, 1H), 3.42 (d, *J* = 11.3 Hz, 1H), 3.14-3.21 (m, 1H), 2.94-3.00 (m, 2H), 2.62 (t, *J* = 10.2 Hz, 2H), 2.50 (td, *J* = 11.2 & 4.0 Hz , 1H), 2.17-2.26 (m, 2H), 2.08 (t, *J* = 9.6 Hz, 1H), 1.61-1.72 (m, 2H), 1.21-1.29 (m, 1H), 1.02 (d, *J* = 6.6 Hz, 3H, 0.99 (d, *J* = 6.6 Hz, 3H); ¹³C NMR (CDCl₃) δ 148.1, 147.59, 128.67, 123.63, 111.85,108.43, 88.70, 84.75, 72,31, 60.64, 58.64, 56.55, 56.05, 52.36, 50.36, 44.19, 37.01, 28.73, 25.89, 24.64, 22.50.

### Example 8 Preparation of Fluorophilic Tetrabenazine Carbinol Compound, Mesylate 19

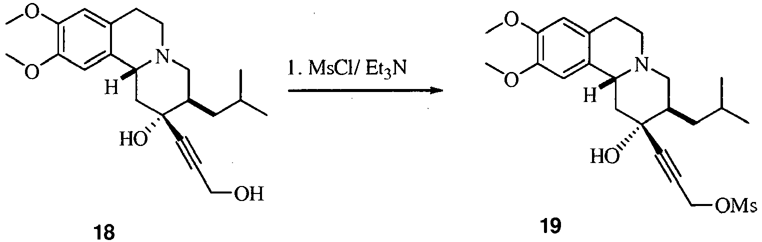

To a 0 °C solution of diol **18** (1 equivalent) in CH₂Cl₂ is added dropwise Et₃N (3 equivalents) followed by methanesulfonyl chloride (MsCl) (1.5 equivalents). The reaction mixture is stirred at 0 °C for about 2 h. After the allotted time the reaction mixture is poured into cold water and layers are separated. The quenched reaction mixture is extracted with three portions of CH₂Cl₂ and the combined organic extracts are dried over anhydrous Na₂SO₄, filtered and concentrated. The residue may be chromatographed on SiO₂ (10% to 70% EtOAc in hexane) to provide the product mesylate **19.**

### Example 9 Preparation of PET Imaging Agent 20

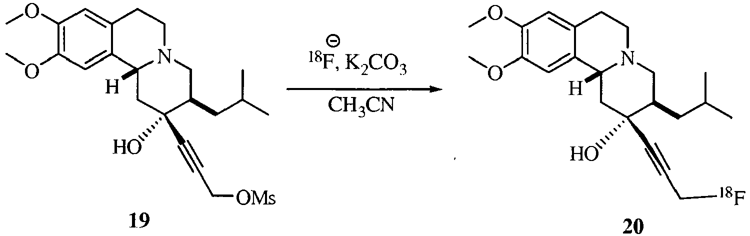

To a Teflon-lined reaction vial contained in a shielded hood and fitted with a nitrogen purge inlet and magnetic spin bar, is added about 1 milliliter of an aqueous acetonitrile solution F-18 fluoride ion, potassium carbonate (about 1 mg), and Kryptofix 221 (about 10 mg). The vial is heated at 100°C under a stream of nitrogen to effect the azeotropic removal of water. Additional dry acetonitrile (1 mL) is added and evaporated. This azeotropic drying protocol is repeated three times. After the final evaporation step a mixture of dimethyl formamide and acetonitrile (about 1 mL) containing the fluorophilic tetrabenazine carbinol compound, mesylate **20,** (2 mg) is added and the vial is sealed. The reaction mixture is stirred and heated at 100°C for 10 minutes and then is cooled to room temperature. The product mixture comprising the starting mesylate **19** and the product F-18 labeled fluoroalkyl tetrabenazine carbinol compound **20** is diluted with water (10 mL) and applied to a Sep-Pak cartridge. The cartridge is then washed with water (3x) to remove unreacted fluoride ion and other water soluble components of the product mixture. The radiolabled fluoroalkyl tetrabenazine carbinol compound **20** and starting mesylate **19** are then eluted from the cartridge with acetonitrile. Most of the acetonitrile is then evaporated and the residue is dissolved in aqueous acetonitrile and subjected to preparative reverse phase HPLC to afford an aqueous formulation comprising PET imaging agent **20**.

### Method 6 Reduction of (+)-tetrabenazine 9 To a Diasteromeric Mixture of Dihydrotetrabenazine Compounds 21 and 22

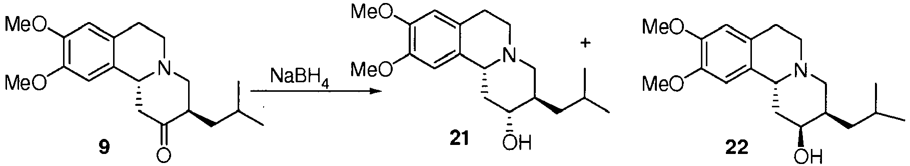

To a 0.11 M solution of (+)-TBZ (9) in ethanol at 0 °C was added NaBH₄ (2.85 eq). The reaction mixture was allowed to stir for 60 min. at room temperature. The solvent was carefully removed under reduced pressure, and the residue was taken up in dichloromethane and washed with three portions of saturated aqueous K₂CO₃. The aqueous washings were back extracted with two portions of dichloromethane. The combined organic extracts were dried (MgSO₄), filtered, and concentrated under reduced pressure to provide a colorless oil that crystallized on standing under high vacuum. Purification of the crude product was achieved by chromatography on SiO₂ (2.5-5% MeOH-CH₂Cl₂, elution was observed at 285 nm) UV active fractions were reanalyzed by TLC. Two products, **21** and **22,** were isolated from this procedure. The major product **21** was a colorless solid 74%: [α]²⁶_{D} +48 (c 0.30, CH₂Cl₂) ¹H NMR (CD₂Cl₂) δ 0.93 (d, *J* = 6.6 Hz, 3H), 0.95 (d, *J* = 6.6 Hz, 3H), 1.04 (ddd, *J* = 14.6, 8.7, 4.3 Hz, 1H), 1.42 (dd, *J* = 20.2, 11.4 Hz, 1H), 1.59 (ddd, *J* = 13.7, 9.6, 3.3 Hz, 1H), 1.64-1.78 (m, 2H), 1.96 (apparent t, *J*= 11.4 Hz, 1H), 2.27 (br s, 1H), 2.40-2.48 (m, 1H), 2.54 (ddd, *J* = 12.3, 3.7, 2.3 Hz, 1H), 2.60-2.67 (m, 1H), 2.95-3.09 (m, 3H), 3.11 (apparent d, *J*= 11.1 Hz, 1H), 3.35 (ddd, *J*= 10.4, 10.4, 4.5 Hz, 1H), 3.80-3.81 (m, 6H), 6.60 (s, 1H), 6.69 (s, 1H); ¹³C NMR (CD₂Cl₂) δ 21.61, 24.02, 25.33, 29.30, 39.68, 40.81, 41.58, 51.83, 55.74, 55.91, 60.02, 60.92, 74.32, 108.42, 111.73, 126.68, 129.76, 147.35, 147.61; HRMS-(ESI+) calcd for (C₁₉H₂₉NO₃ + H) [M+H]⁺ 320.2226, found 320.2242. The minor product 22 was a yellow oil 4%: ¹H NMR (CD₂Cl₂) δ 0.94 (d, *J* = 6.6 Hz, 3H), 0.96 (d, *J* = 6.6 Hz, 3H), 1.13-1.20 (m, 1H), 1.24-1.34 (m, 2H), 1.60-1.77 (m, 2H), 1.89-2.00 (m, 1H) 2.36-2.44 (m, 2H), 2.53 (ddd, *J* = 10.5, 10.5, 3.8 Hz, 1H), 2.58-2.70 (m, 2H), 2.91-2.98 (m, 1H), 2.98-3.09 (m, 1H), 3.48 (apparent d, *J* = 11.6 Hz, 1H), 3.80-3.82 (apparent s, 6H), 4.07 (apparent d, *J* = 3.1Hz, 1H), 6.60 (s, 1H), 6.68 (s, 1H); ¹³C NMR (CD₂Cl₂) δ 22.74, 22.81, 24.87, 29.30, 37.83, 38.87, 39.42, 52.44, 55.76, 55.96, 56.32, 56.43, 67.88, 108.45, 111.78, 127.18, 130.38, 147.30, 147.54.

### Measurement of Blinding Affinity of Fluoroalkyl Tetrabenazine Carbinol Compounds to VMAT-2

VMAT-2 binding affinities were measured for fluoroalkyl tetrabenazine carbinol compounds **16** and **17** provided by the present invention. VMAT-2 binding affinity measurements were carried out by Novascreen Biosciences Corporation (Hanover, Maryland, USA) using protocol Cat. No. 100-0751. Novascreen, Inc. is a commercial provider of biological assays for the pharmaceutical industry. Binding affinity data are presented in Table 11 and illustrate very high binding affinity for the fluoroalkyl tetrabenazine carbinol compounds of the present invention (compounds 16 and **17)** relative to a reserpine control (Comparative Example 1) and a dihydrotetrabenazine (DTBZ) control (Comparative Example 2). The data obtained for fluoroalkyl tetrabenazine carbinol compounds **16** and **17** reveal an unexpected tolerance of fluoroalkyl substitution at ring position-2, which combines a change in the size and lipophilicity of the group at ring position-2 with the uncertainty which arises whenever a hydrogen in a biologically active molecule is replaced by fluorine. In addition, the binding constants Ki expressed in nano-molar (nM) concentration units indicate a very high affinity of the fluoroalkyl tetrabenazine carbinol compounds of the present invention for the VMAT-2 biomarker.

**Table 11 VMAT-2 Binding Affinity of Fluoroalkyl Tetrabenazine Carbinol Compounds 16 and 17**

| Example No. | Compound No. | Structure | Ki (nM) |
|---|---|---|---|
| Example 5 | 16 | 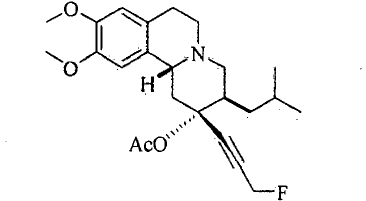 | 19 |
| Example 6 | 17 | 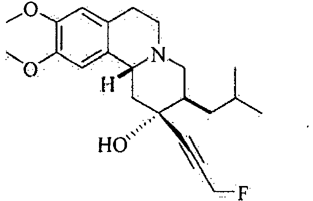 | 19 |
| Comparative Example 1 | Reserpine 23 | 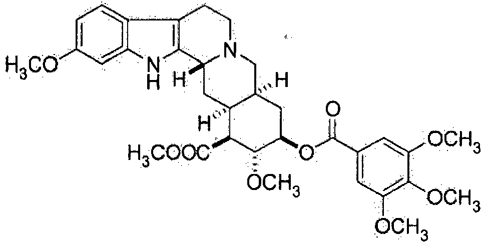 | 162* |
| Comparative Example 2 | DTBZ 21 | 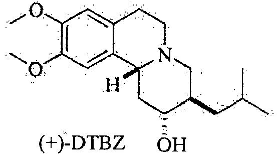 | 3 |

| | | | |
|---|---|---|---|
| * Average of two Ki values obatined for reserpine 70 nM and 254 nM | | | |

## Claims

1. A fluoroalkyl tetrabenazine carbinol compound having structure I: 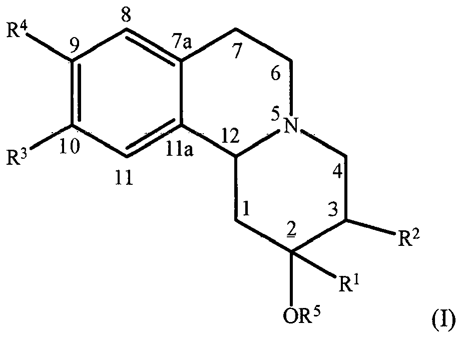
wherein R¹ is selected from C₁₋₆ fluoroalkyl, C₂₋₆ fluoroalkenyl, C₂₋₆ fluoroalkynyl C₁₋₆ fluoroalkoxy(C₁₋₆ alkyl), C₁₋₆ fluorohaloalkyl, C₁₋₆ fluorohydroxyalkyl, and C₁₋₆ fluoroalkylcarbonyl(C₁₋₆ alkyl);
R² is selected from C₁₋₆ alkyl and C₃₋₈ cycloalkyl;
R³ and R⁴ are each independently selected from C₁₋₆ alkyl and C₁₋₆ alkoxy; and
R⁵ is selected from hydrogen, (C₁₋₆ alkyl)carbonyl, and phenyl.

2. The fluoroalkyl tetrabenazine carbinol compound according to claim 1, which is enantiomerically enriched.

3. The enantiomerically enriched fluoroalkyl tetrabenazine carbinol compound according to claim 2 comprising a principal component enantiomer having structure II 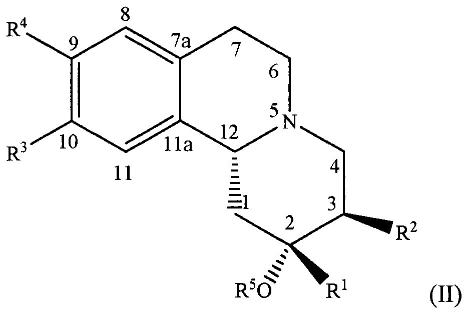 wherein R¹, R², R³, R⁴ and R⁵ are as defined in claim 1.

4. A fluoroalkyl tetrabenazine carbinol compound according to claim 1 having structure IV: 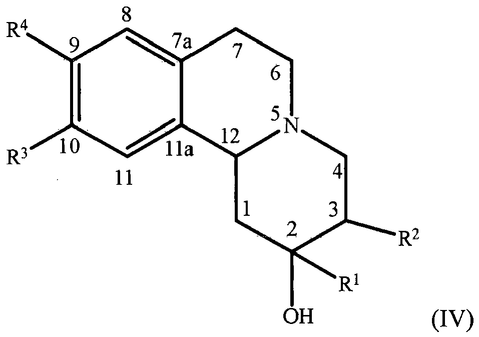 wherein R¹, R², R³ and R⁴ are as defined in claim 1

5. A fluoroalkyl tetrabenazine carbinol compound according to claim 4 which is enantiomerically enriched and comprising a principal component enantiomer having structure V:

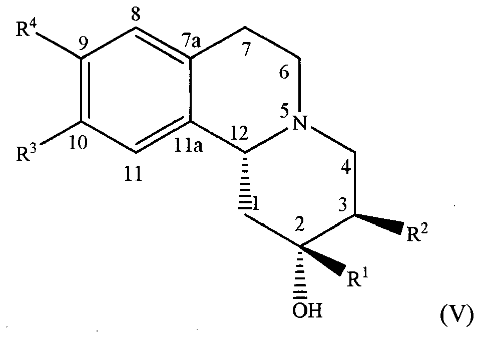

6. The enantiomerically enriched fluoroalkyl tetrabenazine carbinol compound according to claim 3 or claim 5, which is at least 80 % enantiomerically enriched.

7. A compound according to any of claims 1 to 3 wherein -OR⁵ is an ester moiety.

8. A compound according to any of claims 1 to 3 having structure:

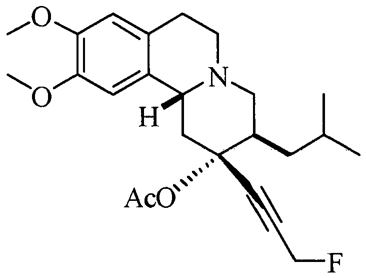

9. A compound according to any of claims 1 to 3 having structure:

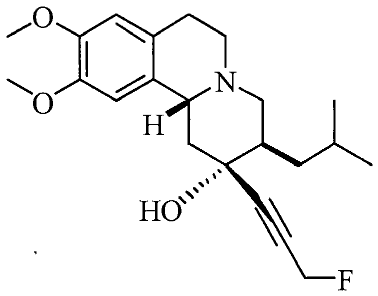

10. A compound according to any of claims 1 to 9, comprising a fluorine-18 atom or a fluorine-19 atom.

11. A salt of a compound according to any of claims 1 to 10.

12. A pharmaceutical formulation comprising a compound according to any one of claims 1 to 11 and a pharmaceutically acceptable excipient.

13. A compound according to any one of claims 1 to 11 for use in medicine.

14. A fluorine-18 labeled compound according to claim 10 , or a salt thereof for use in *in vivo* PET imaging.

## Patentansprüche

1. Fluoralkyltetrabenazincarbinolverbindung mit der Struktur I: 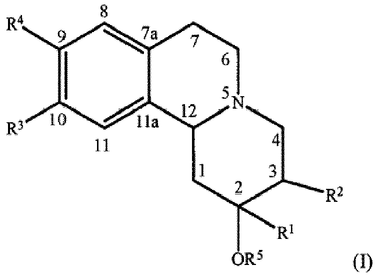
wobei R¹ ausgewählt ist aus C₁₋₆-Fluoralkyl, C₂₋₆-Fluoralkenyl, C₂₋₆-Fluoralkynyl, C₁₋₆-Fluoralkoxy(C₁₋₆-alkyl), C₁₋₆-Fluorhaloalkyl, C₁₋₆-Fluorhydroxyalkyl und C₁₋₆-Fluoralkyl-carbonyl(C₁₋₆-alkyl);
R² ausgewählt ist aus C₁₋₆-Alkyl und C₃₋₈-Cycloalkyl;
R³ und R⁴ jeweils unabhängig ausgewählt sind aus C₁₋₆-Alkyl und C₁₋₆-Alkoxy; und
R⁵ ausgewählt ist aus Wasserstoff, (C₁₋₆-Alkyl)carbonyl und Phenyl.

2. Fluoralkyltetrabenazincarbinolverbindung nach Anspruch 1, welche enantiomerisch angereichert ist.

3. Enantiomerisch angereicherte Fluoralkyltetrabenazincarbinolverbindung nach Anspruch 2, enthaltend ein Hauptkomponenten-Enantiomer mit der Struktur II: 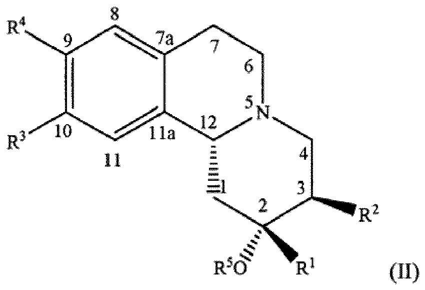 wobei R¹, R², R³, R⁴ und R⁵ der Definition in Anspruch 1 entsprechen.

4. Fluoralkyltetrabenazincarbinolverbindung nach Anspruch 1 mit der Struktur IV: 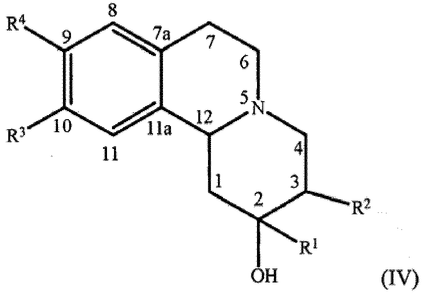 wobei R¹, R², R³ und R⁴ der Definition in Anspruch 1 entsprechen.

5. Fluoralkyltetrabenazincarbinolverbindung nach Anspruch 4, welche enantiomerisch angereichert ist und ein Hauptkomponenten-Enantiomer mit der Struktur V enthält:

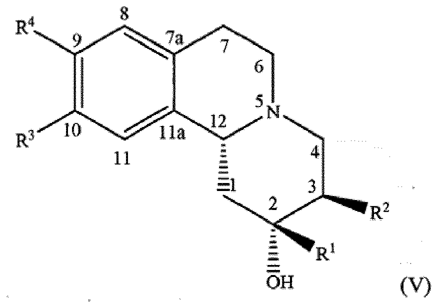

6. Enantiomerisch angereicherte Fluoralkyltetrabenazincarbinolverbindung nach Anspruch 3 oder Anspruch 5, welche mindestens 80 % enantiomerisch angereichert ist.

7. Verbindung nach einem der Ansprüche 1 bis 3, wobei -OR⁵ ein Esteranteil ist.

8. Verbindung nach einem der Ansprüche 1 bis 3 mit der Struktur:

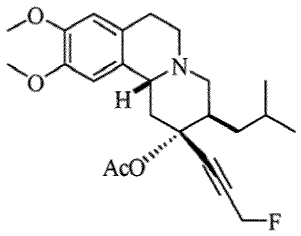

9. Verbindung nach einem der Ansprüche 1 bis 3 mit der Struktur:

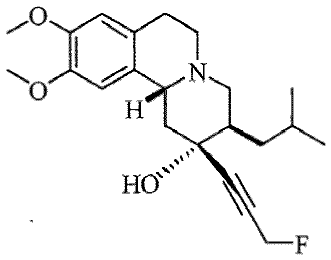

10. Verbindung nach einem der Ansprüche 1 bis 9, enthaltend ein Fluor-18-Atom oder ein Fluor-19-Atom.

11. Salz einer Verbindung nach einem der Ansprüche 1 bis 10.

12. Pharmazeutische Formulierung, die eine Verbindung nach einem der Ansprüche 1 bis 11 und einen pharmazeutisch akzeptablen Arzneistoffträger enthält.

13. Verbindung nach einem der Ansprüche 1 bis 11 zur medizinischen Verwendung.

14. Fluor-18-markierte Verbindung nach Anspruch 10 oder deren Salz zur Verwendung bei In-vivo-PET-Abbildungsverfahren.

## Revendications

1. Composé de carbinol de fluoroalkyltétrabénazine de structure I : 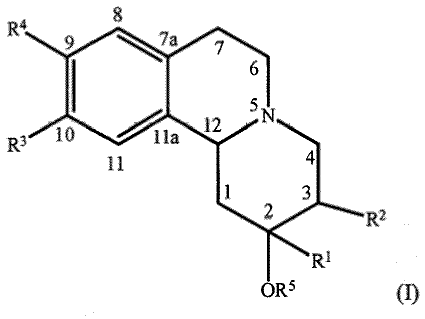
dans laquelle R¹ est choisi parmi un groupement fluoroalkyle en C₁-C₆, fluoroalcényle en C₂-C₆, fluoroalcynyle en C₂-C₆, fluoroalcoxy(C₁-C₆)alkyle en C₁-C₆, fluorohaloalkyle en C₁-C₆, fluoro-hydroxyalkyle en C₁-C₆ et fluoroalkylcarbonyl-(C₁₋C₆)alkyle en C₁₋C₆ ;
R² est choisi parmi un groupement alkyle en C₁-C₆ et un groupement cycloalkyle en C₃-C₈ ;
R³ et R⁴ sont chacun indépendamment choisis parmi un groupement alkyle en C₁-C₆ et un groupement alcoxy en C₁-C₆ ; et
R⁵ est choisi parmi l'hydrogène, un groupement alkyl(C₁-C₆)carbonyle et un groupement phényle.

2. Composé de carbinol de fluoroalkyltétrabénazine selon la revendication 1, qui est enrichi en énantiomères.

3. Composé de carbinol de fluoroalkyltétrabénazine enrichi en énantiomères selon la revendication 2, comprenant un énantiomère comme composant principal, de structure II : 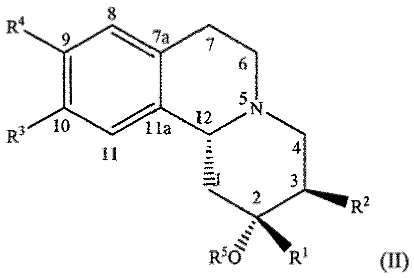 dans laquelle R¹, R², R³, R⁴ et R⁵ sont tels que définis dans la revendication 1.

4. Composé de carbinol de fluroalkyltétrabénazine selon la revendication 1, de structure IV : 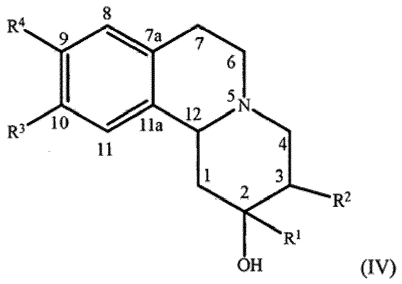 dans laquelle R¹, R², R³ et R⁴ sont tels que définis dans la revendication 1.

5. Composé de carbinol de fluoroalkyltétrabénazine selon la revendication 4, qui est enrichi en énantiomères et comprend un énantiomère comme composant principal, de structure V :

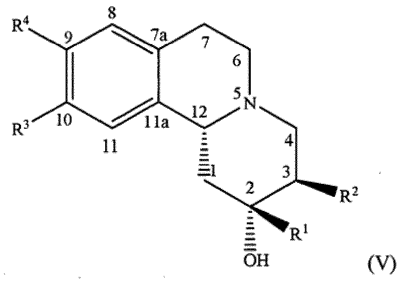

6. Composé de carbinol de fluoroalkyltétrabénazine enrichi en énantiomères selon la revendication 3 ou la revendication 5, qui est enrichi en énantiomères au moins à 80 %.

7. Composé selon l'une quelconque des revendications 1 à 3, dans lequel -OR⁵ est un fragment ester.

8. Composé selon l'une quelconque des revendications 1 à 3, de structure :

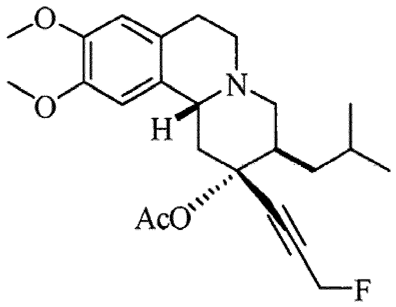

9. Composé selon l'une quelconque des revendications 1 à 3, de structure :

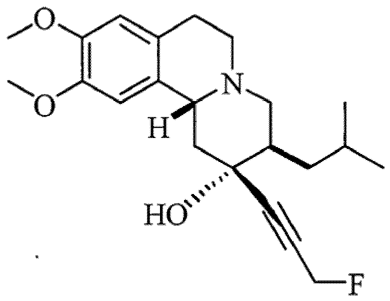

10. Composé selon l'une quelconque des revendications 1 à 9, comprenant un atome de fluor-18 ou un atome de fluor-19.

11. Sel d'un composé selon l'une quelconque des revendications 1 à 10.

12. Formulation pharmaceutique comprenant un composé selon l'une quelconque des revendications 1 à 11 et un excipient pharmaceutiquement acceptable.

13. Composé selon l'une quelconque des revendications 1 à 11 pour un usage en médecine.

14. Composé marqué au fluor-18 selon la revendication 10 ou son sel pour un usage en imagerie PET *in vivo.*
